(19) Europäisches Patentamt
European Patent Office
Office européen des brevets

(11) Veröffentlichungsnummer: **0 054 865**
**B1**

(12) # EUROPÄISCHE PATENTSCHRIFT

(45) Veröffentlichungstag der Patentschrift: **26.02.86**

(51) Int. Cl.⁴: **C 07 D 233/60**, C 07 D 249/08, A 61 K 31/415, A 61 K 31/41

(21) Anmeldenummer: **81110365.4**

(22) Anmeldetag: **11.12.81**

(54) **Substituierte 1-Azolyl-butan-2-one, Verfahren zu ihrer Herstellung und ihre Verwendung als Fungizide sowie als Zwischenprodukte.**

(30) Priorität: **20.12.80 DE 3048266**

(43) Veröffentlichungstag der Anmeldung: **30.06.82 Patentblatt 82/26**

(45) Bekanntmachung des Hinweises auf die Patenterteilung: **26.02.86 Patentblatt 86/9**

(84) Benannte Vertragsstaaten: **AT BE CH DE FR GB IT LI NL SE**

(56) Entgegenhaltungen:
**EP - A - 0 009 707**
**EP - A - 0 031 911**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Büchel, Karl Heinz, Prof.Dr., Dabringhausener Strasse 42, D-5093 Burscheid (DE)**
Erfinder: **Elbe, Hans-Ludwig, Dr., Dasnöckel 59, D-5600 Wuppertal 11 (DE)**
Erfinder: **Kraatz, Udo, Dr., Körnerstrasse 6, D-5090 Leverkusen 1 (DE)**
Erfinder: **Regel, Erik, Ing., Bergerheide 72a, D-5600 Wuppertal 1 (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., D-5653 Leichlingen 1 (DE)**

**EP 0 054 865 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue substituierte 1-Azolyl-butan-2-one, mehrere Verfahren zu ihrer Herstellung sowie ihre Verwendung als Fungizide und als Zwischenprodukte für die Synthese von weiteren Pflanzenschutzmitteln.

Es ist bereits bekannt geworden, daß bestimmte Triazol-yl-keto—Derivate, wie z.B. 3,3-Dimethyl-1-(1,2,4-triazol-1-yl)-butan-2-on, ω-(1,2,4-Triazol-1-yl)-2,4-dichloracetophenon oder 1-(4-Chlorphenyl)-4,4-dimethyl-2-(1,2,4-triazol-1-yl)-pentan-3-on, allgemein gute fungizide Wirksamkeit besitzen (vergleiche DE-OS 24 31 407 und DE-OS 27 34 426). Die Wirkung dieser Triazol-Derivate ist jedoch in bestimmten Indikationsbereichen, insbesondere bei niedrigen Aufwandmengen und -konzentrationen, nicht immer ganz befriedigend.

Es wurden neue substituierte 1-Azolyl-butan-2-one der allgemeinen Formel

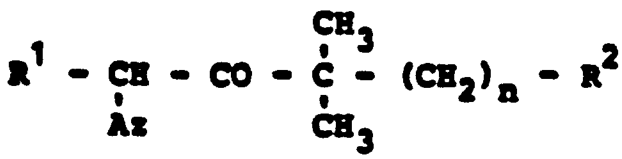

(I)

in welcher

5 Az für 1,2,4-Triazol-1-yl und -4-yl oder Imidazol-1-yl steht,

$R^1$ für Wasserstoff, für Alkyl mit 1 bis 12 und Alkenyl und Alkinyl mit jeweils 2 bis 12 Kohlenstoffatomen steht, für gegebenenfalls durch Alkyl mit 1 bis 4 Kohlenstoffatomen substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im Alkylteil steht, sowie für gegebenenfalls substituiertes Phenoxyalkyl mit 1 bis 4 Kohlenstoffatomen im Alkylteil oder gegebenenfalls substituiertes Phenylalkyl mit 1 bis 4 Kohlenstoffatomen im Alkylteil steht, wobei als Phenyl-Substituenten jeweils zu nennen sind: Halogen; Alkyl, Alkoxy und Alkylthio mit jeweils 1 bis 6 Kohlenstoffatomen; Cyclohexyl; Dialkylamino mit jeweils 1 bis 4 Kohlenstoffatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkylteil; sowie gegebenenfalls durch Halogen substituiertes Phenyl und Phenoxy; sowie die Gruppierung -CO-$NR^6R^7$;

n für die Zahlen 0 oder 1 steht,

$R^2$ für Cyano oder die Gruppierungen -X-$R^3$ und -CO-$NR^4R^5$ steht, sowie für den Fall, daß nicht gleichzeitig n für die Zahl 0, $R^1$ für Wasserstoff und Az für 1,2,4-Triazolyl stehen, noch zusätzlich für gegebenenfalls substituiertes Aryl mit 6 bis 10 Kohlenstoffatomen steht, wobei als Substituenten die bei $R^1$ bereits genannten Arylsubstituenten infrage kommen, sowie für Alkoxycarbonyl mit 1 bis 4 Kohlenstoffatomen im Alkylteil steht; wobei

X für Sauerstoff, Schwefel, die SO- oder $SO_2$-Gruppe steht,

$R^3$ für Alkyl mit 1 bis 6 Kohlenstoffatomen, Halogenalkyl mit 1 bis 4 Kohlenstoffatomen und 1 bis 5 gleichen oder verschiedenen Halogenatomen steht, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im Alkylteil steht, wobei als Substituenten die bei $R^1$ bereits genannten Arylsubstituenten infrage kommen;

$R^4$ für Wasserstoff, Alkyl mit 1 bis 4 Kohlenstoffatomen oder gegebenenfalls substituiertes Aryl mit 1 bis 10 Kohlenstoffatomen steht, wobei als Substituenten die bei $R^1$ bereits genannten Arylsubstituenten infrage kommen;

$R^5$ für Wasserstoff oder Alkyl mit 1 bis 4 Kohlenstoffatomen steht; und

$R^6$ und $R^7$ für Wasserstoff, für Alkyl mit 1 bis 4 Kohlenstoffatomen, für gegebenenfalls durch Halogen und Alkyl mit 1 bis 4 Kohlenstoffatomen substituiertes phenyl stehen, oder beide zusammen mit dem angrenzenden Stickstoffatom für ein gesättigtes 5- oder 6-gliedriges Ringsystem stehen, welches Stickstoff oder Sauerstoff als zusätzliche Heteroatome enthalten kann;

sowie deren pflanzenverträglichen Säureadditions-Salze und Metallsalz-Komplexe gefunden.

Weiterhin wurde gefunden, daß man die substituierten 1-Azolyl-butan-2-one der Formel (I) sowie deren pflanzenverträglichen Säureadditions-Salze und Metallsalz-Komplexe erhält, wenn man

a) Halogenketone der Formel

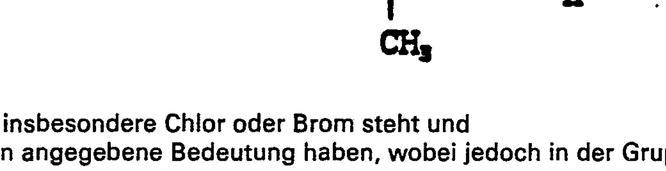

(II)

in welcher

Hal für Halogen, insbesondere Chlor oder Brom steht und

$R^2$ und n die oben angegebene Bedeutung haben, wobei jedoch in der Gruppierung -X-$R^3$ der Substituent X

nur für Sauerstoff oder Schwefel steht,

mit Azolen der Formel

H - Az (III)

in welcher

Az die oben angegebene Bedeutung hat,

in Gegenwart eines Verdünnungsmittels und in Gegenwart eines Säurebindemittels umsetzt; und gegebenenfalls

b) die so erhaltenen Verbindungen der Formel

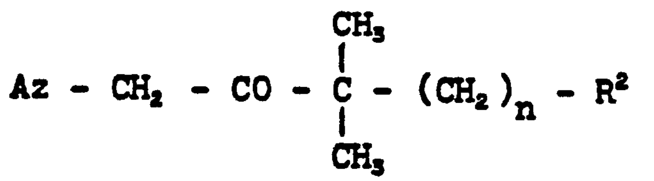

(Ia)

in welcher

AZ, n und $R^2$ die oben angegebene Bedeutung haben,

mit einem Alkylierungsmittel der Formel

$R^1$ - Z (IV)

in welcher

$R^1$ die oben angegebene Bedeutung hat und

Z für eine elektronenanziehende Abgangsgruppierung steht,

in Gegenwart einer Base und in Gegenwart eines organischen Verdünnungsmittels, oder in einem wässrigorganischen Zweiphasensystem in Gegenwart eines Phasentransferkatalysators, umsetzt; und gegebenenfalls

c) die nach den Verfahren (a) und (b) erhaltenen Verbindungen der Formel

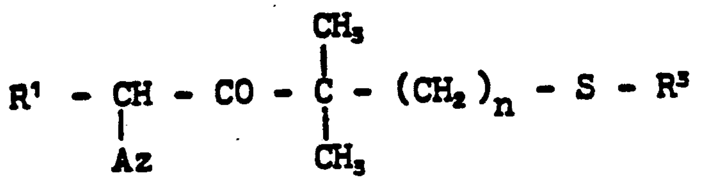

(Ib)

in welcher

Az,$R^i$,n

und $R^3$ die oben angegebene Bedeutung haben,

nach bekannten Methoden in üblicher Weise oxidiert.

An die so erhaltenen Verbindungen der Formel (I) kann gegebenenfalls anschließend eine Säure oder ein Metallsalz addiert werden. In manchen Fällen erweist es sich als vorteilhaft, die Verbindungen der Formel (I) über ihre Salze in reiner Form zu erhalten.

Die neuen substituierten 1-Azolyl-butan-2-one der Formel (I) weisen starke fungizide Eigenschaften auf. Dabei zeigen überraschenderweise die erfindungsgemäßen Verbindungen eine bessere fungizide Wirkung als die aus dem Stand der Technik bekannten Triazolyl-keto-Derivate 3,3-Dimethyl-1-(1,2,4-triazol-1-yl)-butan-2-on, ω-(1,2,4-Triazol-1-yl)-2,4-dichloracetphenon und 1-(4-Chlorphenyl)-4,4- dimethyl-2-(1,2,4-triazol-1-yl)-pentan-3-on, die chemisch und wirkungsmäßig ähnliche Verbindungen sind.

Außerdem sind die neuen substituierten 1-Azolyl-butan-2-one der Formel (I) interessante Zwischenprodukte zur Herstellung von weiteren pflanzenschutz-Wirkstoffen. So kann z.B. - wie noch ausgeführt wird - die Ketogruppe zu einer -CH(OH)-Gruppe reduziert werden. Ferner können durch entsprechende Umsetzungen funktionelle Derivate der Ketogruppe erhalten werden, wie z.B. Oxime und Oximether, Hydrazone und Ketale.

Die erfindungsgemäßen Stoffe stellen somit eine wertvolle Bereicherung der Technik dar.

Bevorzugt sind diejenigen Verbindungen der formel (I), in der

$\underline{R}^1$ für geradkettiges oder verzweigtes Alkyl mit 1 bis 6 Kohlenstoffatomen, Alkenyl und Alkinyl mit jeweils 2 bis 6 Kohlenstoffatomen, gegebenenfalls durch Methyl substituiertes Cyclohexyl und Cyclohexylmethyl, sowie für gegebenenfalls substituiertes phenoxyalkyl und gegebenenfalls substituiertes phenylalkyl mit jeweils 1 bis 2 Kohlenstoffatomen im Alkylteil steht, wobei als phenyl-Substituenten gsnannt seien: Fluor, Chlor, Methyl, Ethyl, Isopropyl, tert.-Butyl, Dimethylamino, Methoxy, Methylthio, Cyclohexyl, Trifluormethyl, Trifluormethoxy, Trifluormethylthio, Nitro, Cyano, sowie gegebenenfalls durch Fluor und Chlor substituiertes phenyl und phenoxy, sowie die Morpholinocarbonyl-, Phenylaminocarbonyl-, Chlorphenylaminocarbonyl- und Dibutyleminocarbonyl-Gruppe;

$\underline{R}^2$ für Cyano; die Gruppierungen -X-$R^3$ und -CO-N$R^4R^5$ steht; sowie für den Fall, daß nicht gleichzeitig n für die Zahl 0, $R^1$ für Wasserstoff und Az für 1,2,4-Triazolyl stehen, noch für gegebenenfalls substituiertes phenyl steht, wobei als Substituenten die bei $R^1$ bereits genannten Phenylsubstituenten infrage kommen, und schließlich für Methoxycarbonyl, Ethoxycarbonyl und Isopropoxycarbonyl steht;

R³ für geradkettiges oder verzweigtes Alkyl mit 1 bis 4 Kohlenstoffatomen oder gegebenenfalls substituiertes phenyl und Benzyl steht, wobei als Substituenten an den jeweiligen phenylresten die bei R¹ bereits genannten phenylsubstituenten infrage kommen;

R⁴ für Wasserstoff, Methyl, Ethyl, Isopropyl oder gegebenenfalls substituiertes phenyl steht, wobei als Substituenten Halogen und Alkyl mit 1 bis 4 Kohlenstoffatomen infrage kommen;

R⁵ für Wasserstoff, Methyl, Ethyl oder Isopropyl steht; und

Az,X und der Index n die in der Erfindungsdefinition angegebene Bedeutung haben.

Im einzelnen seien außer den bei den Herstellungsbeispielen genannten Verbindungen folgende Verbindungen der allgemeinen Formel (I) genannt (wobei Az für 1,2,4-Triazol-1-yl oder Imidazol-1-yl steht):

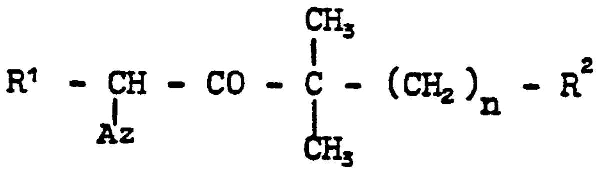

(I)

| $R^1$ | n | $R^2$ |
|---|---|---|
| H | 1 | 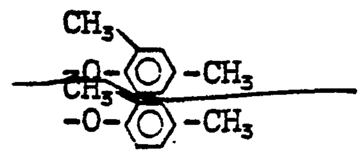 |
| $C_2H_5$ | 1 | |
| $C_4H_9$ | 1 | 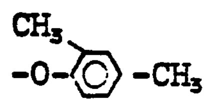 |
| 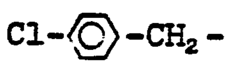 | 1 | 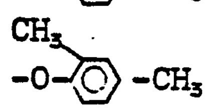 |
| 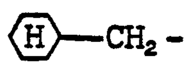 | 1 | 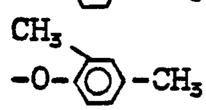 |
| H | 1 | 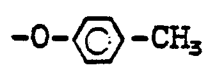 |
| $C_2H_5$ | 1 | 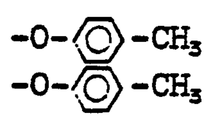 |
| $C_4H_9$ | 1 | |
| 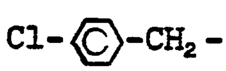 | 1 | 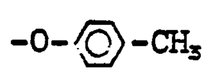 |
| 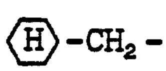 | 1 | 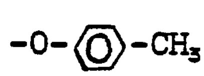 |
| H | 1 | 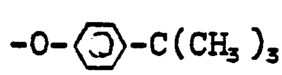 |
| $C_2H_5$ | 1 | 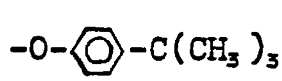 |

| $R^1$ | n | $R^2$ |
|---|---|---|
| $C_4H_9$ | 1 | 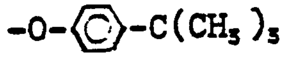 |
| 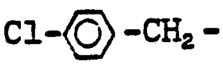 | 1 | 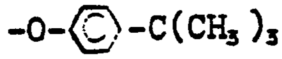 |
| 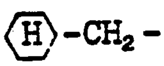 | 1 | 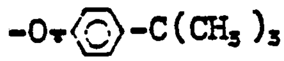 |
| H | 1 | 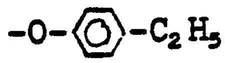 |
| $C_2H_5$ | 1 | 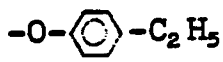 |
| $C_4H_9$ | 1 | 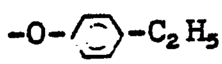 |
| 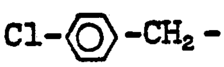 | 1 | 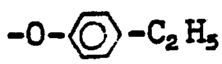 |
| 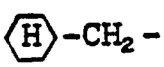 | 1 | 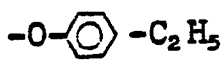 |
| H | 1 | 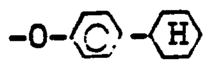 |
| $C_2H_5$ | 1 | 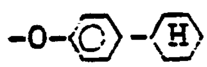 |
| $C_4H_9$ | 1 | 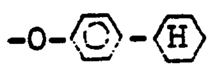 |
| 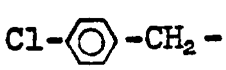 | 1 | 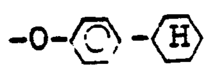 |
| 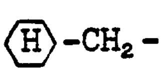 | 1 | 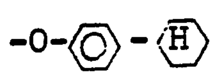 |
| H | 1 | 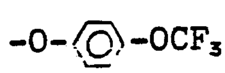 |
| $C_2H_5$ | 1 | 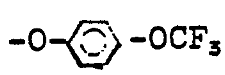 |
| $C_4H_9$ | 1 | 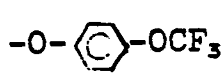 |

| $R^1$ | n | $R^2$ |
|---|---|---|
| 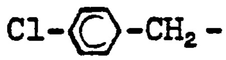 | 1 | 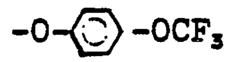 |
| 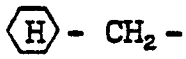 | 1 | 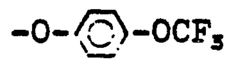 |
| H | 1 | 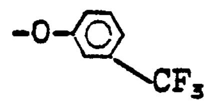 |
| $C_2H_5$ | 1 | 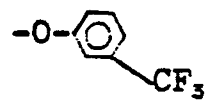 |
| $C_4H_9$ | 1 | 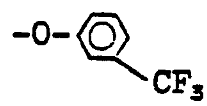 |
| 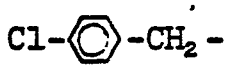 | 1 | 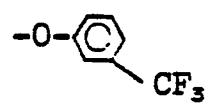 |
| 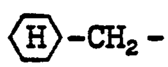 | 1 | 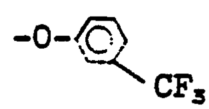 |
| H | 1 | 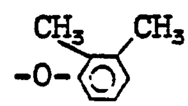 |
| $C_2H_5$ | 1 | 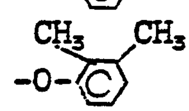 |
| $C_4H_9$ | 1 | 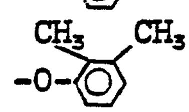 |
| 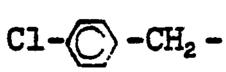 | 1 | 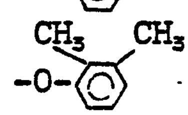 |
| 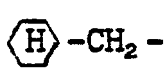 | 1 | 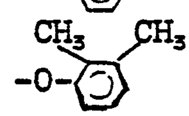 |

| $R^1$ | n | $R^2$ |
|---|---|---|
| H | 1 | 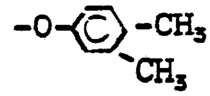 |
| $C_2H_5$ | 1 | 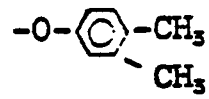 |
| $C_4H_9$ | 1 | 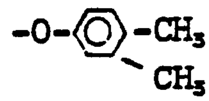 |
| 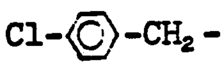 | 1 | 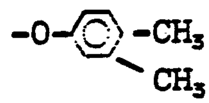 |
| 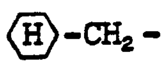 | 1 | 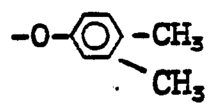 |
| H | 1 | 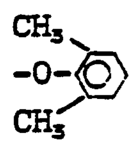 |
| $C_2H_5$ | 1 | 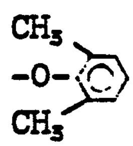 |
| $C_4H_9$ | 1 | 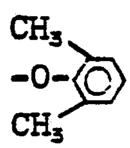 |
| 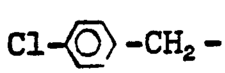 | 1 | 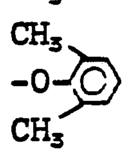 |
| 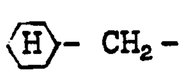 | 1 | 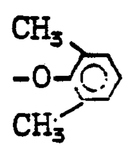 |
| H | 1 | 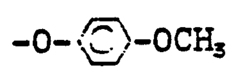 |
| $C_2H_5$ | 1 | 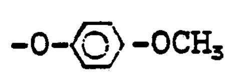 |

| $R^1$ | n | $R^2$ |
|---|---|---|
| $C_4H_9$ | 1 | 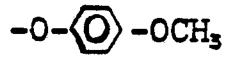 |
| 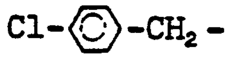 | 1 | 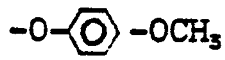 |
| 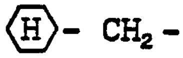 | 1 | 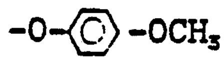 |
| H | 1 | 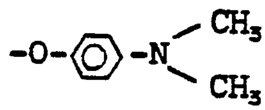 |
| $C_2H_5$ | 1 | 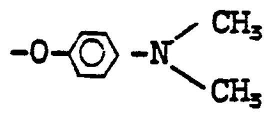 |
| $C_4H_9$ | 1 | 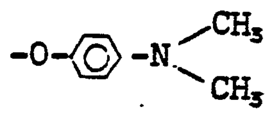 |
| 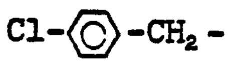 | 1 | 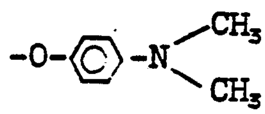 |
| 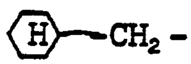 | 1 | 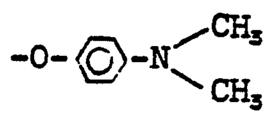 |
| H | 1 | 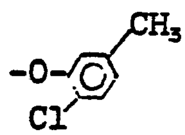 |
| $C_2H_5$ | 1 | 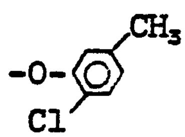 |
| $C_4H_9$ | 1 | 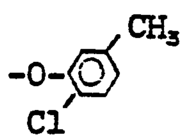 |
| 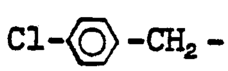 | 1 | 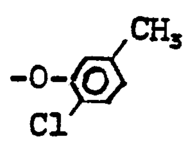 |
| 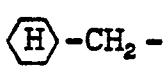 | 1 | 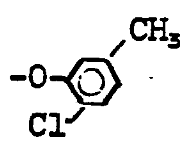 |

| $R^1$ | n | $R^2$ |
|---|---|---|
| H | 1 | 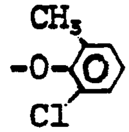 |
| $C_2H_5$ | 1 | 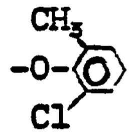 |
| $C_4H_9$ | 1 | 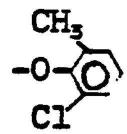 |
| 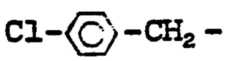 | 1 | 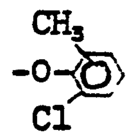 |
| 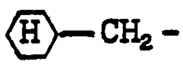 | 1 | 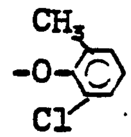 |
| H | 1 | 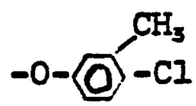 |
| $C_2H_5$ | 1 | 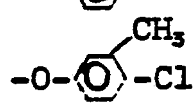 |
| $C_4H_9$ | 1 | 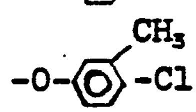 |
| 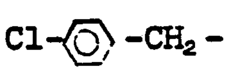 | 1 | 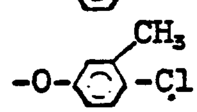 |
| 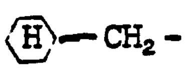 | 1 | 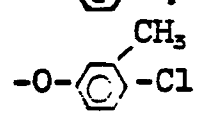 |
| H | 1 | 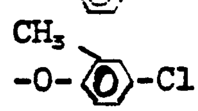 |
| $C_2H_5$ | 1 | 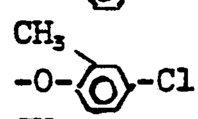 |
| $C_4H_9$ | 1 | 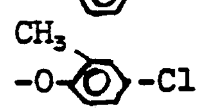 |
| 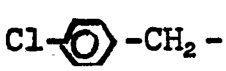 | 1 | 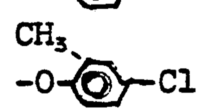 |

| $R^1$ | n | $R^2$ |
|---|---|---|
| 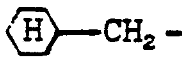 | 1 | 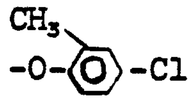 |
| H | 1 | 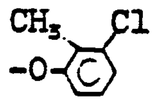 |
| $C_2H_5$ | 1 | 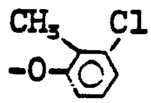 |
| $C_4H_9$ | 1 | 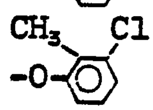 |
| 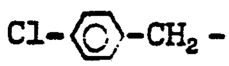 | 1 | 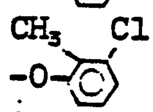 |
| 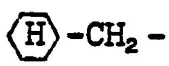 | 1 | 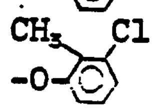 |
| H | 1 | 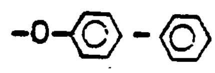 |
| $C_2H_5$ | 1 | 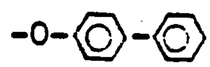 |
| $C_4H_9$ | 1 | 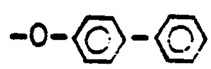 |
| 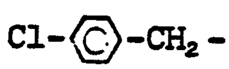 | 1 | 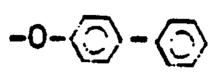 |
| 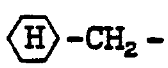 | 1 | 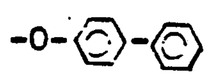 |
| H | 1 | 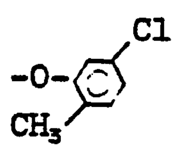 |
| $C_2H_5$ | 1 | 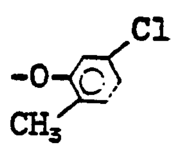 |
| $C_4H_9$ | 1 | 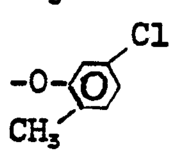 |

| $R^1$ | n | $R^2$ |
|---|---|---|
| 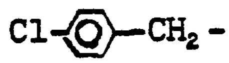 | 1 | 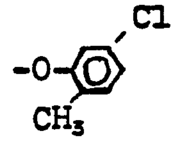 |
| 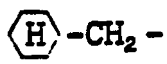 | 1 | 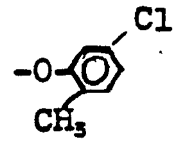 |
| H | 1 | 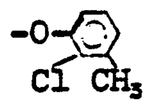 |
| $C_2H_5$ | 1 | 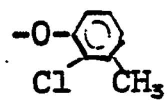 |
| $C_4H_9$ | 1 | 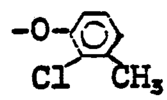 |
| 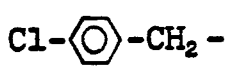 | 1 | 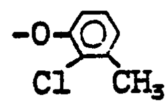 |
| 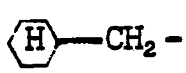 | 1 | 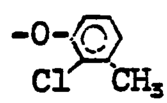 |
| H | 1 | 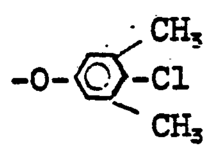 |
| $C_2H_5$ | 1 | 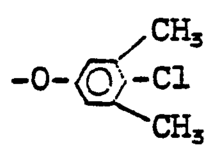 |
| $C_4H_9$ | 1 | 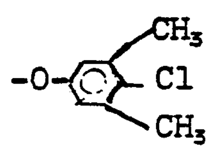 |
| 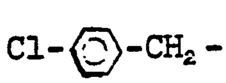 | 1 | 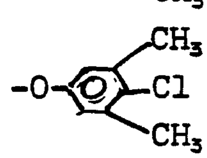 |

| $R^1$ | n | $R^2$ |
|---|---|---|
| 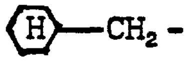 | 1 | 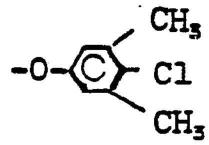 |
| H | 1 | 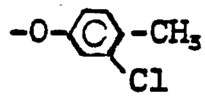 |
| $C_2H_5$ | 1 | 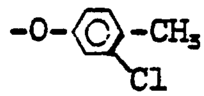 |
| $C_4H_9$ | 1 | 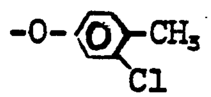 |
| 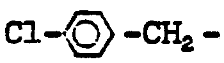 | 1 | 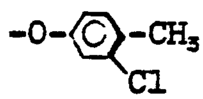 |
| 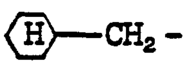 | 1 | 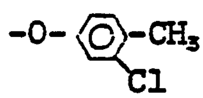 |
| H | 1 | 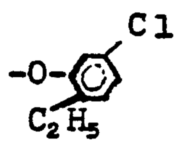 |
| $C_2H_5$ | 1 | 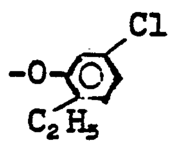 |
| $C_4H_9$ | 1 | 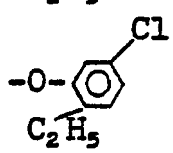 |
| 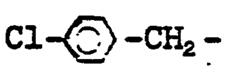 | 1 | 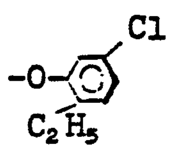 |
| 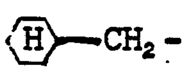 | 1 | 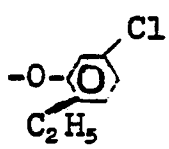 |

| $R^1$ | n | $R^2$ |
|---|---|---|
| H | 1 | 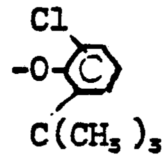 |
| $C_2H_5$ | 1 | 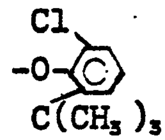 |
| $C_4H_9$ | 1 | 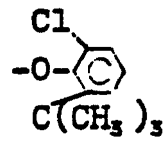 |
| 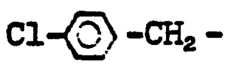 | 1 | 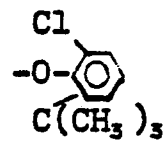 |
| 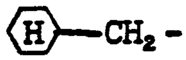 | 1 | 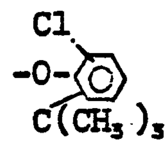 |
| H | 1 | 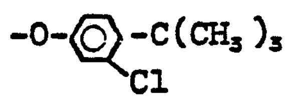 |
| $C_2H_5$ | 1 | 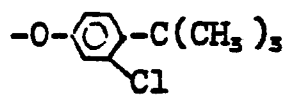 |
| $C_4H_9$ | 1 | 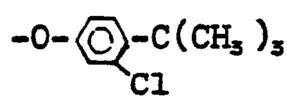 |
| 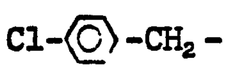 | 1 | 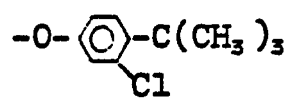 |
| 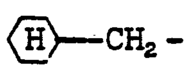 | 1 | 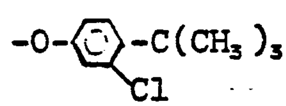 |

| $R^1$ | n | $R^2$ |
|---|---|---|
| H | 1 | 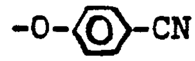 |
| $C_2H_5$ | 1 | 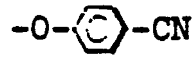 |
| $C_4H_9$ | 1 | 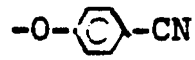 |
| 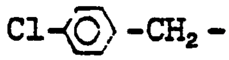 | 1 | 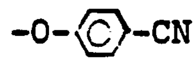 |
| 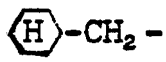 | 1 | 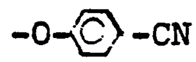 |
| H | 1 | 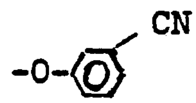 |
| $C_2H_5$ | 1 | 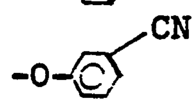 |
| $C_4H_9$ | 1 | 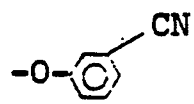 |
| 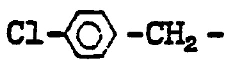 | 1 | 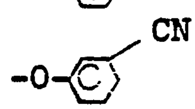 |
| 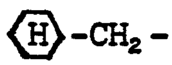 | 1 | 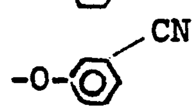 |
| H | 1 | 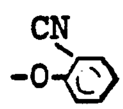 |
| $C_2H_5$ | 1 | 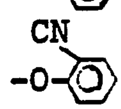 |
| $C_4H_9$ | 1 | 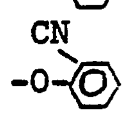 |
| 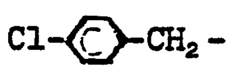 | 1 | 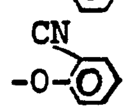 |
| 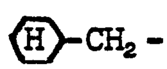 | 1 | 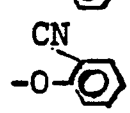 |
| H | 1 | 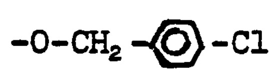 |

| $R^1$ | n | $R^2$ |
|---|---|---|
| $C_2H_5$ | 1 | $-O-CH_2-$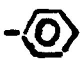$-Cl$ |
| $C_4H_9$ | 1 | $-O-CH_2-$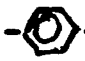$-Cl$ |
| $Cl-$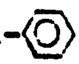$-CH_2-$ | 1 | $-O-CH_2-$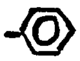$-Cl$ |
| 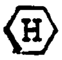$-CH_2-$ | 1 | $-O-CH_2-$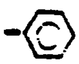$-Cl$ |
| H | 1 | $-O-CH_2-$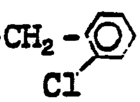 |
| $C_2H_5$ | 1 | $-O-CH_2-$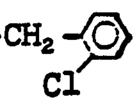 |
| $C_4H_9$ | 1 | $-O-CH_2-$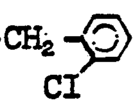 |
| $Cl-$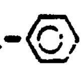$-CH_2-$ | 1 | $-O-CH_2-$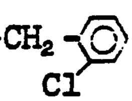 |
| 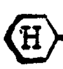$-CH_2-$ | 1 | $-O-CH_2-$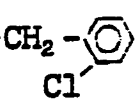 |
| H | 1 | $-O-CH_2-$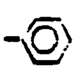 |
| $C_2H_5$ | 1 | $-O-CH_2-$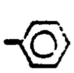 |
| $C_4H_9$ | 1 | $-O-CH_2-$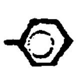 |
| $Cl-$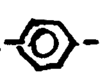$-CH_2-$ | 1 | $-O-CH_2-$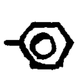 |
| 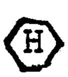$-CH_2-$ | 1 | $-O-CH_2-$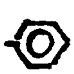 |

| $R^1$ | n | $R^2$ |
|---|---|---|
| H | 1 | 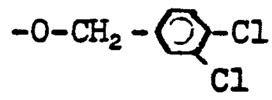 |
| $C_2H_5$ | 1 | 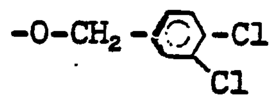 |
| $C_4H_9$ | 1 | 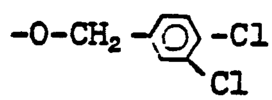 |
| 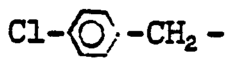 | 1 | 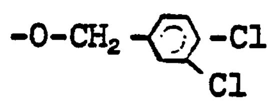 |
| 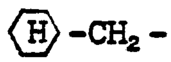 | 1 | 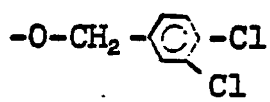 |
| H | 1 | 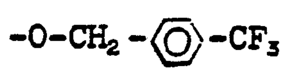 |
| $C_2H_5$ | 1 | 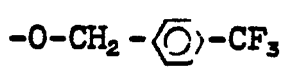 |
| $C_4H_9$ | 1 | 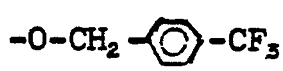 |
| 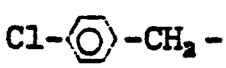 | 1 | 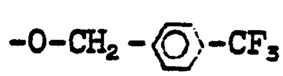 |
| 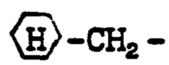 | 1 | 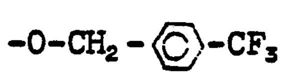 |
| H | 1 | 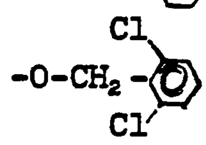 |
| $C_2H_5$ | 1 | 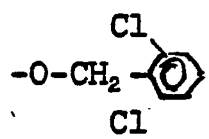 |

| $R^1$ | n | $R^2$ |
|---|---|---|
| $C_4H_9$ | 1 | 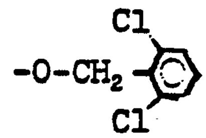 |
| 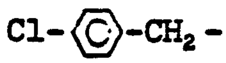 | 1 | 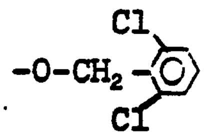 |
| 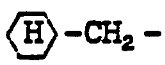 | 1 | 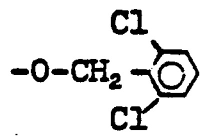 |
| H | 1 | 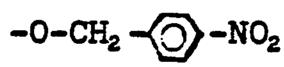 |
| $C_2H_5$ | 1 | 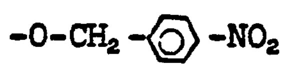 |
| $C_4H_9$ | 1 | 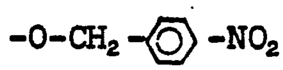 |
| 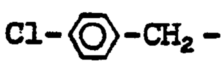 | 1 | 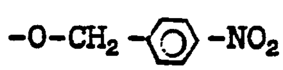 |
| 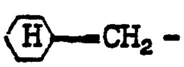 | 1 | 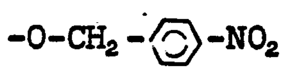 |
| H | 1 | 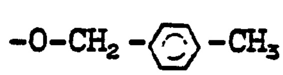 |
| $C_2H_5$ | 1 | 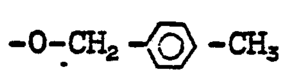 |
| $C_4H_9$ | 1 | 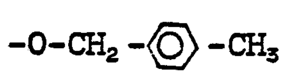 |
| 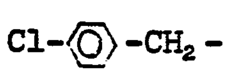 | 1 | 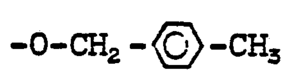 |
| 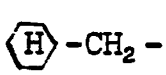 | 1 | 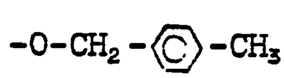 |

| $R^1$ | n | $R^2$ |
|---|---|---|
| H | 1 | 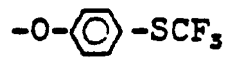 |
| $C_2H_5$ | 1 | 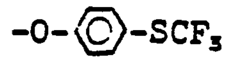 |
| $C_4H_9$ | 1 | 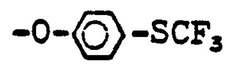 |
| 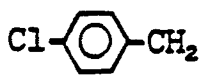 | 1 | 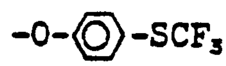 |
| 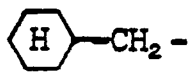 | 1 | 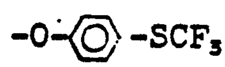 |
| H | 1 | 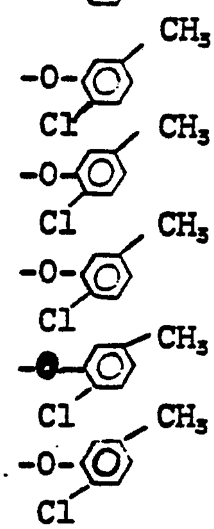 |
| $C_2H_5$ | 1 | |
| $C_4H_9$ | 1 | |
| 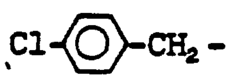 | 1 | |
| 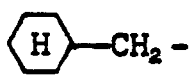 | 1 | |

| $R^1$ | n | $R^2$ |
|---|---|---|
| H | 0 | 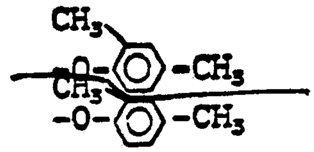 |
| $C_2H_5$ | 0 | |
| $C_4H_9$ | 0 | 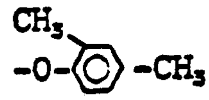 |
| 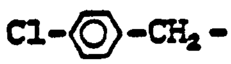 | 0 | 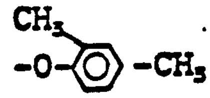 |
| 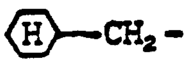 | 0 | 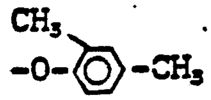 |
| H | 0 | 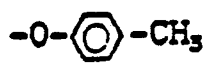 |
| $C_2H_5$ | 0 | 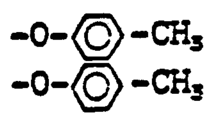 |
| $C_4H_9$ | 0 | |
| 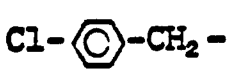 | 0 | 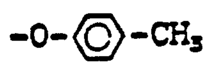 |
| 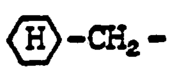 | 0 | 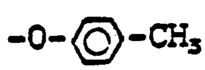 |
| H | 0 | 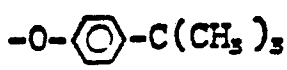 |
| $C_2H_5$ | 0 | 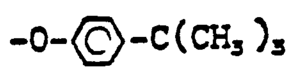 |

| $R^1$ | n | $R^2$ |
|---|---|---|
| $C_4H_9$ | 0 | 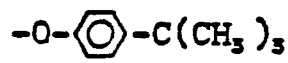 |
| 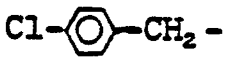 | 0 | 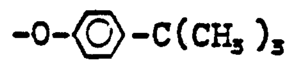 |
| 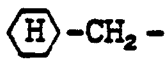 | 0 | 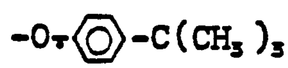 |
| H | 0 | 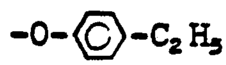 |
| $C_2H_5$ | 0 | 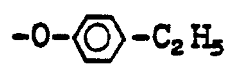 |
| $C_4H_9$ | 0 | 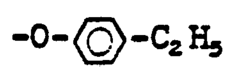 |
| 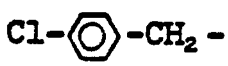 | 0 | 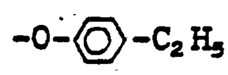 |
| 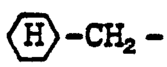 | 0 | 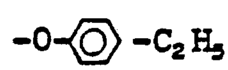 |
| H | 0 | 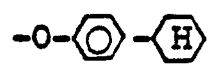 |
| $C_2H_5$ | 0 | 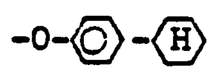 |
| $C_4H_9$ | 0 | 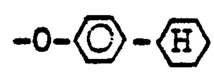 |
| 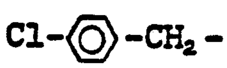 | 0 | 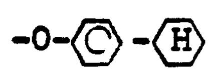 |
| 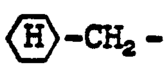 | 0 | 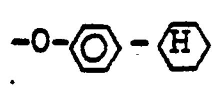 |
| H | 0 | 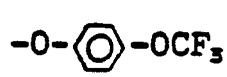 |
| $C_2H_5$ | 0 | 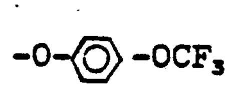 |
| $C_4H_9$ | 0 | 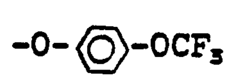 |

| $R^1$ | n | $R^2$ |
|---|---|---|
| 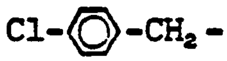 | 0 | 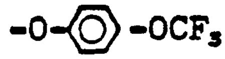 |
| 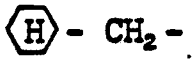 | 0 | 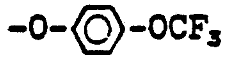 |
| H | 0 | 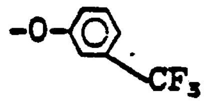 |
| $C_2H_5$ | 0 | 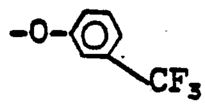 |
| $C_4H_9$ | 0 | 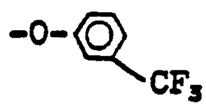 |
| 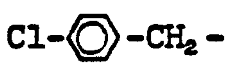 | 0 | 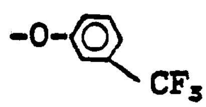 |
| 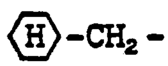 | 0 | 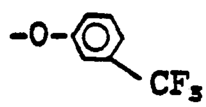 |
| H | 0 | 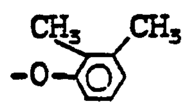 |
| $C_2H_5$ | 0 | 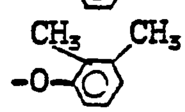 |
| $C_4H_9$ | 0 | 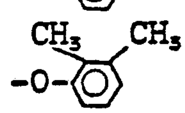 |
| 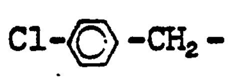 | 0 | 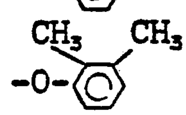 |
| 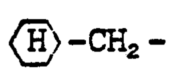 | 0 | 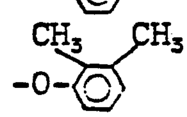 |

| $R^1$ | n | $R^2$ |
|---|---|---|
| H | 0 | 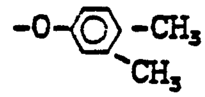 |
| $C_2H_5$ | 0 | 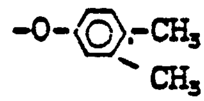 |
| $C_4H_9$ | 0 | 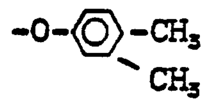 |
| 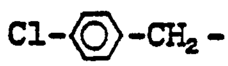 | 0 | 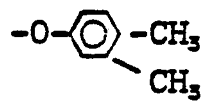 |
| 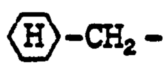 | 0 | 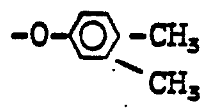 |
| H | 0 | 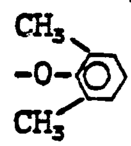 |
| $C_2H_5$ | 0 | 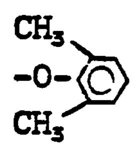 |
| $C_4H_9$ | 0 | 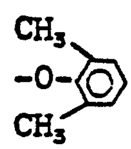 |
| 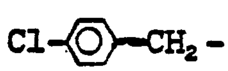 | 0 | 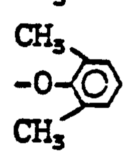 |
| 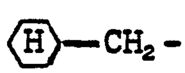 | 0 | 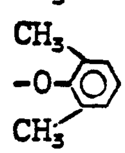 |
| H | 0 | 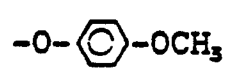 |
| $C_2H_5$ | 0 | 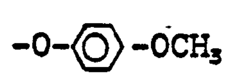 |

| $R^1$ | n | $R^2$ |
|---|---|---|
| $C_4H_9$ | 0 | 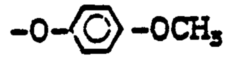 |
| 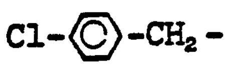 | 0 | 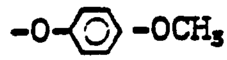 |
| 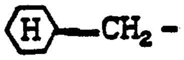 | 0 | 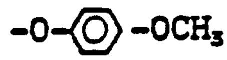 |
| H | 0 | 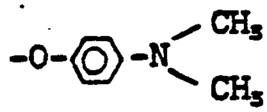 |
| $C_2H_5$ | 0 | 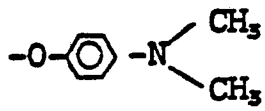 |
| $C_4H_9$ | 0 | 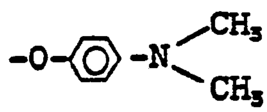 |
| 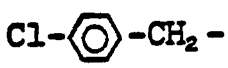 | 0 | 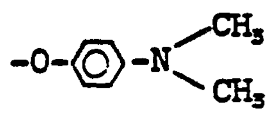 |
| 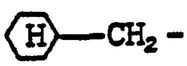 | 0 | 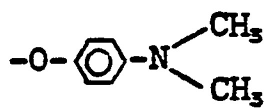 |
| H | 0 | 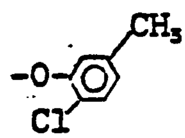 |
| $C_2H_5$ | 0 | 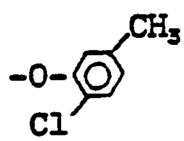 |
| $C_4H_9$ | 0 | 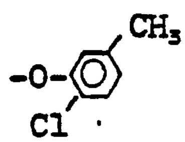 |
| 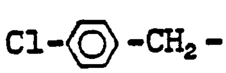 | 0 | 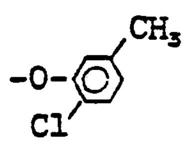 |
| 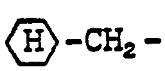 | 0 | 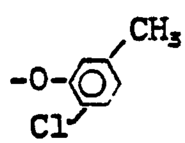 |

| $R^1$ | n | $R^2$ |
|---|---|---|
| H | 0 | 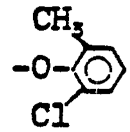 |
| $C_2H_5$ | 0 | 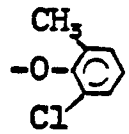 |
| $C_4H_9$ | 0 | 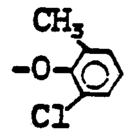 |
| 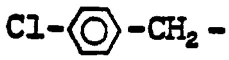 | 0 | 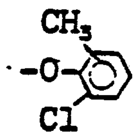 |
| 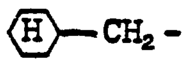 | 0 | 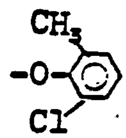 |
| H | 0 | 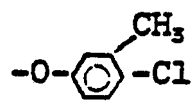 |
| $C_2H_5$ | 0 | 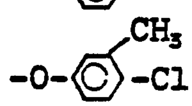 |
| $C_4H_9$ | 0 | 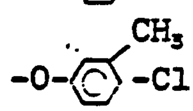 |
| 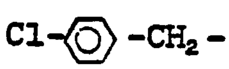 | 0 | 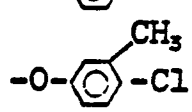 |
| 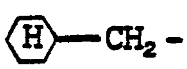 | 0 | 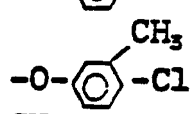 |
| H | 0 | 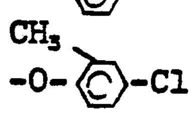 |
| $C_2H_5$ | 0 | 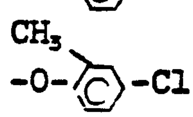 |
| $C_4H_9$ | 0 | 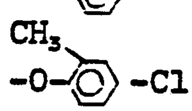 |
| 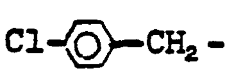 | 0 | 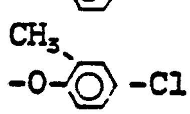 |

| $R^1$ | n | $R^2$ |
|---|---|---|
| 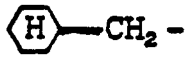 | 0 | 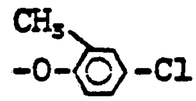 |
| H | 0 | 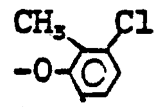 |
| $C_2H_5$ | 0 | 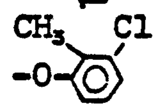 |
| $C_4H_9$ | 0 | 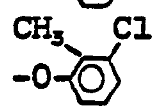 |
| 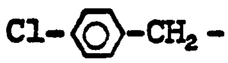 | 0 | 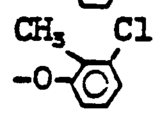 |
| 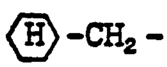 | 0 | 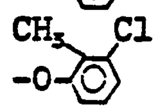 |
| H | 0 | 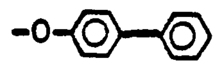 |
| $C_2H_5$ | 0 | 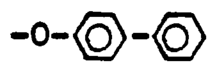 |
| $C_4H_9$ | 0 | 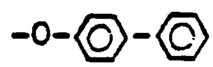 |
| 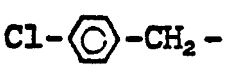 | 0 | 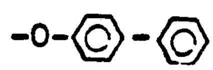 |
| 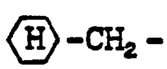 | 0 | 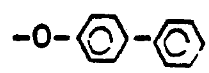 |
| H | 0 | 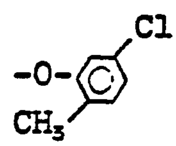 |
| $C_2H_5$ | 0 | 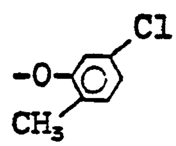 |
| $C_4H_9$ | 0 | 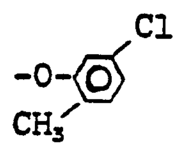 |

| $R^1$ | n | $R^2$ |
|---|---|---|
| 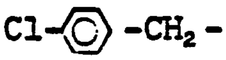 | 0 | 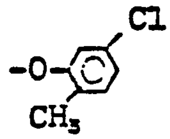 |
| 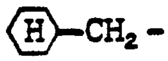 | 0 | 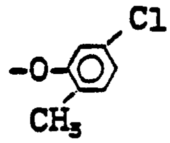 |
| H | 0 | 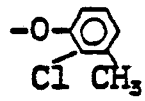 |
| $C_2H_5$ | 0 | 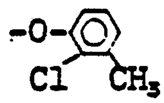 |
| $C_4H_9$ | 0 | 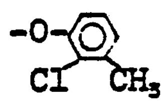 |
| 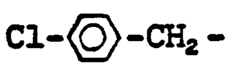 | 0 | 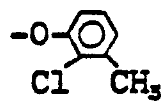 |
| 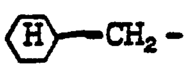 | 0 | 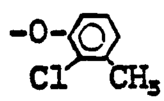 |
| H | 0 | 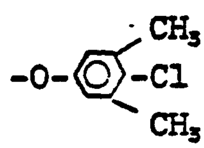 |
| $C_2H_5$ | 0 | 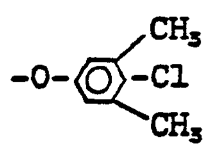 |
| $C_4H_9$ | 0 | 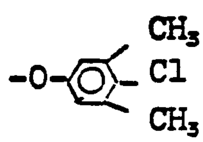 |
| 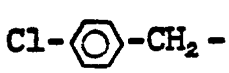 | 0 | 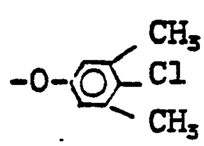 |

| $R^1$ | n | $R^2$ |
|---|---|---|
| 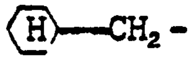 | 0 | 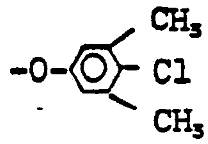 |
| H | 0 | 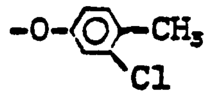 |
| $C_2H_5$ | 0 | 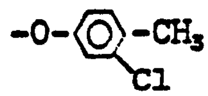 |
| $C_4H_9$ | 0 | 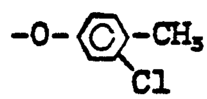 |
| 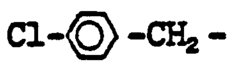 | 0 | 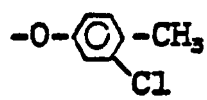 |
| 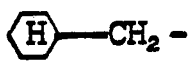 | 0 | 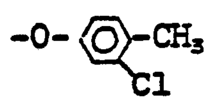 |
| H | 0 | 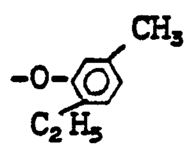 |
| $C_2H_5$ | 0 | 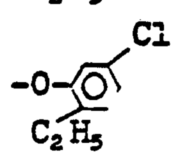 |
| $C_4H_9$ | 0 | 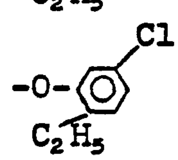 |
| 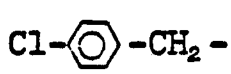 | 0 | 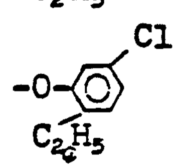 |
| 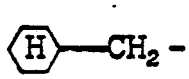 | 0 | 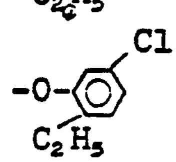 |

| $R^1$ | n | $R^2$ |
|---|---|---|
| H | 0 | 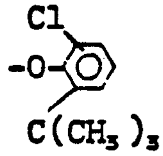 |
| $C_2H_5$ | 0 | 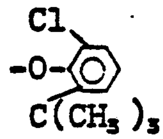 |
| $C_4H_9$ | 0 | 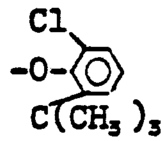 |
| 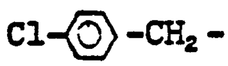 | 0 | 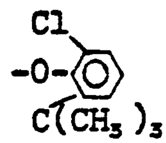 |
| 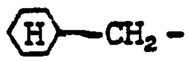 | 0 | 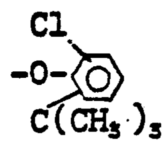 |
| H | 0 | 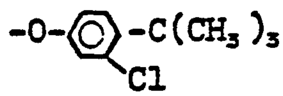 |
| $C_2H_5$ | 0 | 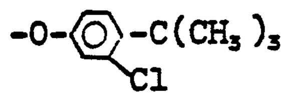 |
| $C_4H_9$ | 0 | 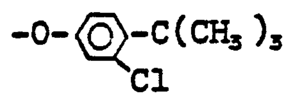 |
| 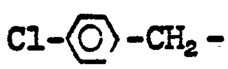 | 0 | 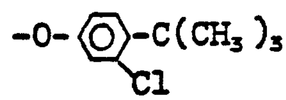 |
| 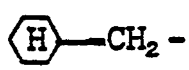 | 0 | 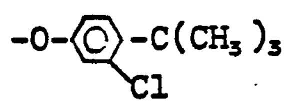 |

| $R^1$ | n | $R^2$ |
|---|---|---|
| H | 0 | 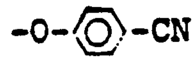 |
| $C_2H_5$ | 0 | 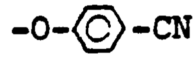 |
| $C_4H_9$ | 0 | 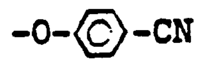 |
| 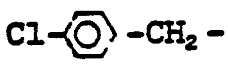 | 0 | 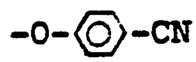 |
| 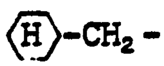 | 0 | 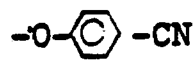 |
| H | 0 | 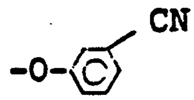 |
| $C_2H_5$ | 0 | 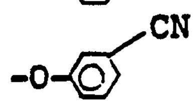 |
| $C_4H_9$ | 0 | 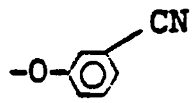 |
| 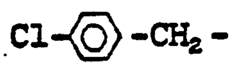 | 0 | 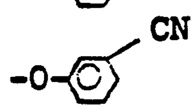 |
| 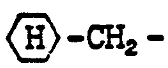 | 0 | 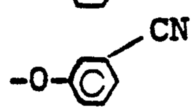 |
| H | 0 | 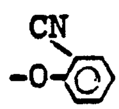 |
| $C_2H_5$ | 0 | 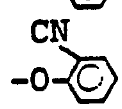 |
| $C_4H_9$ | 0 | 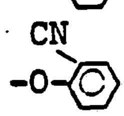 |
| 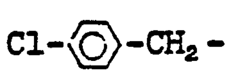 | 0 | 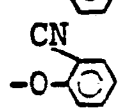 |
| 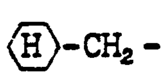 | 0 | 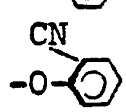 |
| H | 0 | 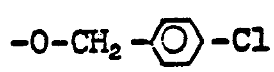 |

| $R^1$ | n | $R^2$ |
|---|---|---|
| $C_2H_5$ | 0 | 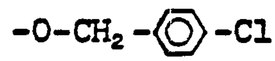 |
| $C_4H_9$ | 0 | 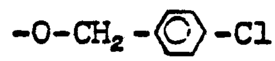 |
| 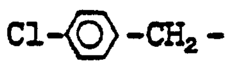 | 0 | 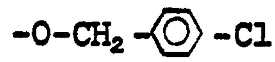 |
| 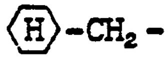 | 0 | 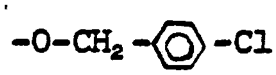 |
| H | 0 | 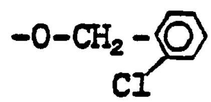 |
| $C_2H_5$ | 0 | 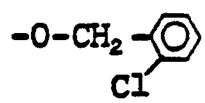 |
| $C_4H_9$ | 0 | 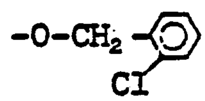 |
| 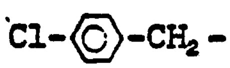 | 0 | 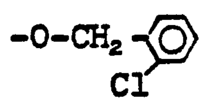 |
| 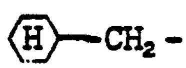 | 0 | 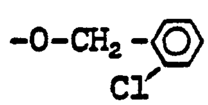 |
| H | 0 | 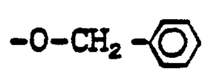 |
| $C_2H_5$ | 0 | 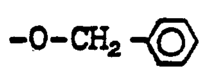 |
| $C_4H_9$ | 0 | 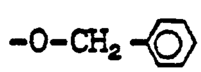 |
| 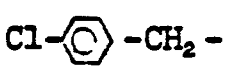 | 0 | 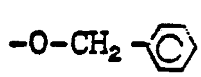 |
| 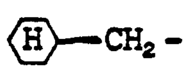 | 0 | 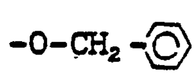 |

| $R^1$ | n | $R^2$ |
|---|---|---|
| H | 0 | 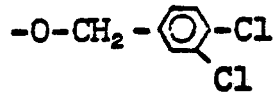 |
| $C_2H_5$ | 0 | 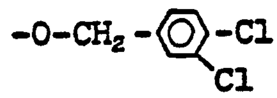 |
| $C_4H_9$ | 0 | 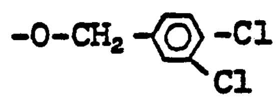 |
| 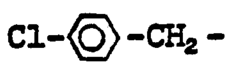 | 0 | 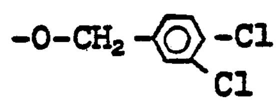 |
| 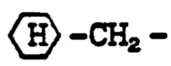 | 0 | 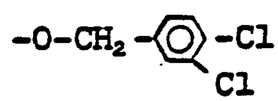 |
| H | 0 | 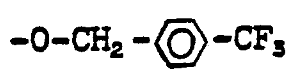 |
| $C_2H_5$ | 0 | 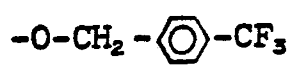 |
| $C_4H_9$ | 0 | 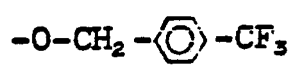 |
| 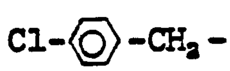 | 0 | 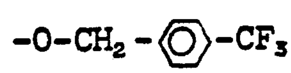 |
| 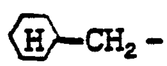 | 0 | 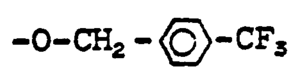 |
| H | 0 | 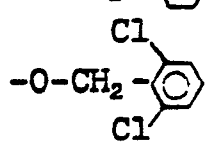 |
| $C_2H_5$ | 0 | 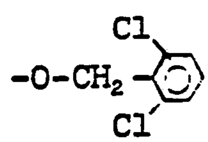 |

| $R^1$ | n | $R^2$ |
|---|---|---|
| $C_4H_9$ | 0 | 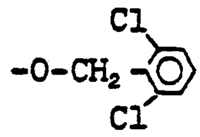 |
| 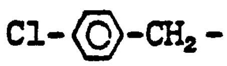 | 0 | 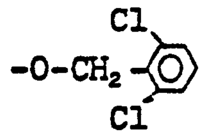 |
| 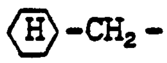 | 0 | 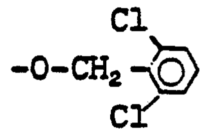 |
| H | 0 | 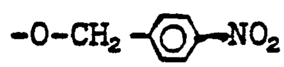 |
| $C_2H_5$ | 0 | 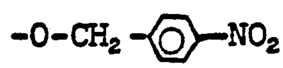 |
| $C_4H_9$ | 0 | 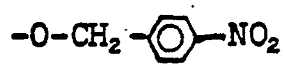 |
| 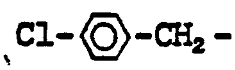 | 0 | 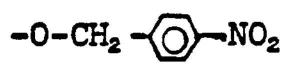 |
| 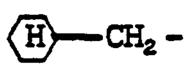 | 0 | 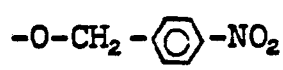 |
| H | 0 | 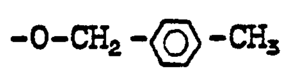 |
| $C_2H_5$ | 0 | 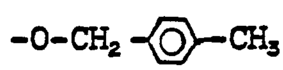 |
| $C_4H_9$ | 0 | 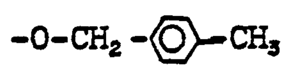 |
| 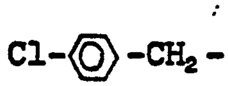 | 0 | 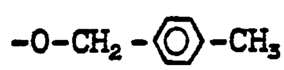 |
| 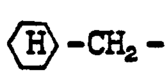 | 0 | 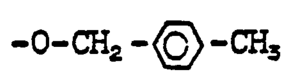 |

| $R^1$ | n | $R^2$ |
|---|---|---|
| H | 0 | 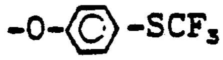 |
| $C_2H_5$ | 0 | 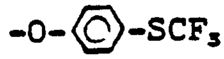 |
| $C_4H_9$ | 0 | 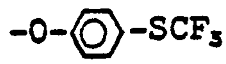 |
| 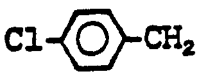 | 0 | 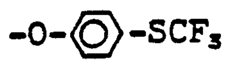 |
| 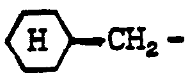 | 0 | 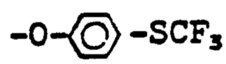 |
| H | 0 | 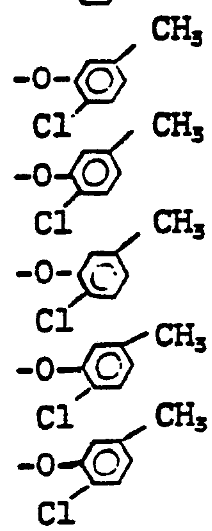 |
| $C_2H_5$ | 0 | |
| $C_4H_9$ | 0 | |
| 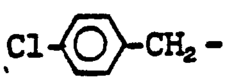 | 0 | |
| 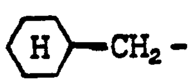 | 0 | |

| $R^1$ | n | $R^2$ |
|---|---|---|
| H | 1 | $-O-C_4H_9$ |
| 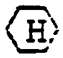 $-CH_2-$ | 1 | $-O-C_4H_9$ |
| $C_2H_5$ | 1 | $-O-C_4H_9$ |
| $C_4H_9$ | 1 | $-O-C_4H_9$ |
| Cl- 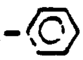 $-CH_2-$ | 1 | $-O-C_4H_9$ |
| $F_3C-$ 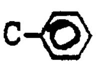 $-CH_2-$ | 0 | $-COOC_2H_5$ |
| $F_3C-$ 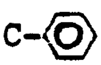 $-CH_2-$ | 0 | $-COOC_3H_7-i$ |
| $F_3C-$ 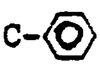 $-CH_2-$ | 0 | $-CON(CH_3)_2$ |
| $F_3C-$ 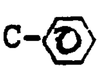 $-CH_2-$ | 0 | -CONH- 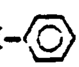 -Cl |
| $F_3CO-$ 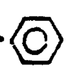 $-CH_2-$ | 0 | $-COOCH_3$ |
| $F_3CO-$ 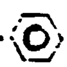 $-CH_2-$ | 0 | -CN |
| H | 0 | -CN |
| $F_3C-$ 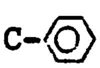 $-CH_2-$ | 0 | - 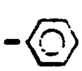 |
| $F_3C-$ 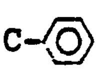 $-CH_2-$ | 0 | - 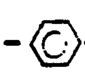 -Cl |
| $F_3C-$ 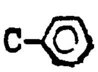 $-CH_2-$ | 0 | - 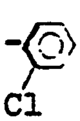 -Cl<br>Cl |
| $F_3C-$ 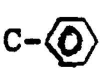 $-CH_2-$ | 0 | - 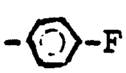 -F |

| $R^1$ | n | $R^2$ |
|---|---|---|
| H | 1 | -S-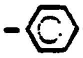-F |
| $-C_4H_9$-n | 1 | -S-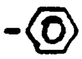-F |
| $-CH_2$-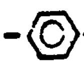-Cl | 1 | -S-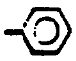-F |
| $-CH_2$-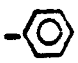$-OCF_3$ | 1 | -S-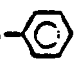-F |
| $-CH_2$-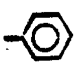$-CF_3$ | 1 | -S-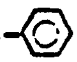-F |
| H | 1 | -S-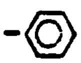$-SCF_3$ |
| $-C_4H_9$-n | 1 | -S-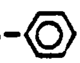$-SCF_3$ |
| $-CH_2$-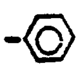-Cl | 1 | -S-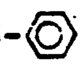$-SCF_3$ |
| $-CH_2$-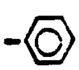$-OCF_3$ | 1 | -S-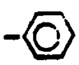$-SCF_3$ |
| $-CH_2$-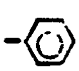$-CF_3$ | 1 | -S-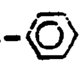$-SCF_3$ |
| H | 1 | -S-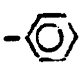$-OCF_3$ |
| $-C_4H_9$-n | 1 | -S-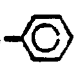$-OCF_3$ |
| $-CH_2$-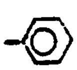-Cl | 1 | -S-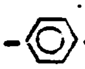$-OCF_3$ |
| $-CH_2$-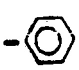$-OCF_3$ | 1 | -S-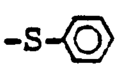$-OCF_3$ |
| $-CH_2$-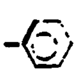$-OCF_3$ | 1 | -S-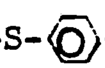$-OCF_3$ |

| $R^1$ | n | $R^2$ |
|---|---|---|
| H | 1 | 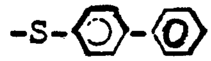 |
| $-C_4H_9$-n | 1 | 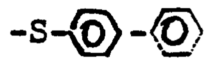 |
| 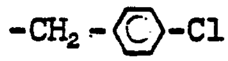 | 1 | 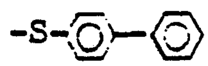 |
| 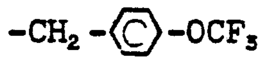 | 1 | 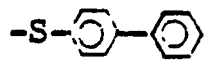 |
| 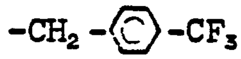 | 1 | 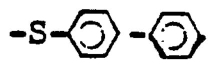 |
| H | 1 | 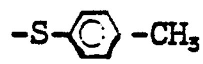 |
| $-C_4H_9$-n | 1 | 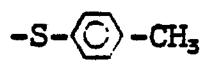 |
| 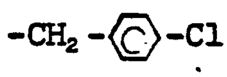 | 1 | 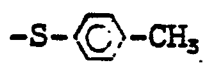 |
| 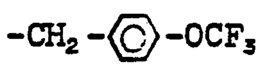 | 1 | 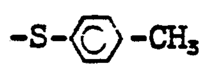 |
| 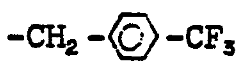 | 1 | 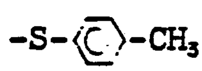 |
| H | 1 | 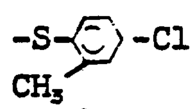 |
| $-C_4H_9$-n | 1 | 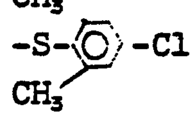 |
| 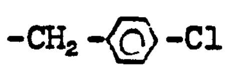 | 1 | 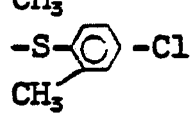 |
| 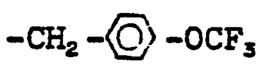 | 1 | 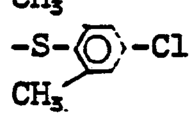 |
| 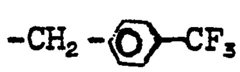 | 1 | 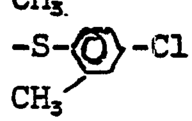 |

| $R^1$ | n | $R^2$ |
|---|---|---|
| H | 1 | 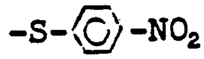 |
| $-C_4H_9$-n | 1 | 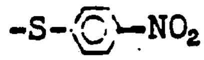 |
| 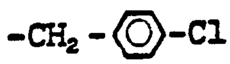 | 1 | 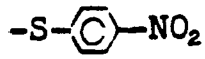 |
| 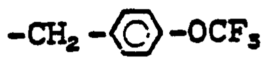 | 1 | 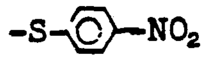 |
| 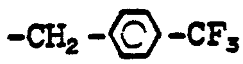 | 1 | 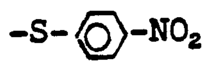 |
| H | 1 | 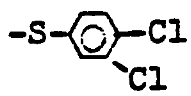 |
| $-C_4H_9$-n | 1 | 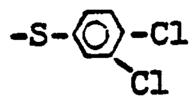 |
| 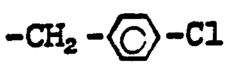 | 1 | 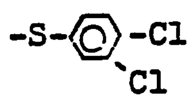 |
| 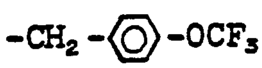 | 1 | 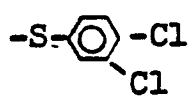 |
| 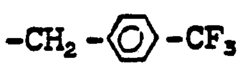 | 1 | 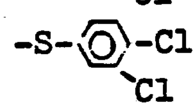 |
| H | 1 | 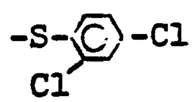 |
| $-C_4H_9$-n | 1 | 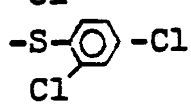 |
| 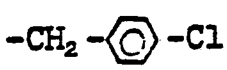 | 1 | 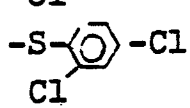 |
| 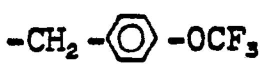 | 1 | 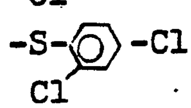 |
| 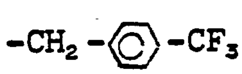 | 1 | 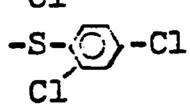 |

| $R^1$ | n | $R^2$ |
|---|---|---|
| H | 1 | 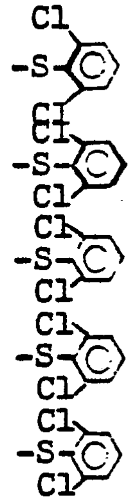 |
| $-C_4H_9-n$ | 1 | |
| $-CH_2-$ 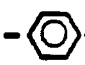 $-Cl$ | 1 | |
| $-CH_2-$ 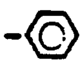 $-OCF_3$ | 1 | |
| $-CH_2-$ 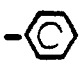 $-CF_3$ | 1 | |
| H | 1 | 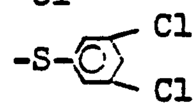 |
| $-C_4H_9-n$ | 1 | 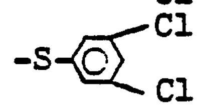 |
| $-CH_2-$ 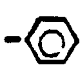 $-Cl$ | 1 | 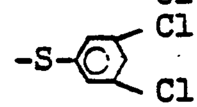 |
| $-CH_2-$ 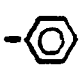 $-OCF_3$ | 1 | 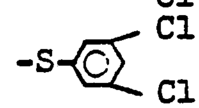 |
| $-CH_2-$ 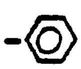 $-CF_3$ | 1 | 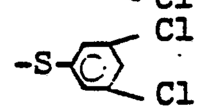 |
| H | 1 | $-S-C_4H_9-n$ |
| $-C_4H_9-n$ | 1 | $-S-C_4H_9-n$ |
| $-CH_2-$ 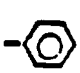 $-Cl$ | 1 | $-S-C_4H_9-n$ |
| $-CH_2-$ 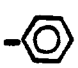 $-OCF_3$ | 1 | $-S-C_4H_9-n$ |
| $-CH_2-$ 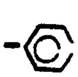 $-CF_3$ | 1 | $-S-C_4H_9-n$ |

| $R^1$ | n | $R^2$ |
|---|---|---|
| H | 1 | $-SCH_3$ |
| $-C_4H_9-n$ | 1 | $-SCH_3$ |
| $-CH_2-$ 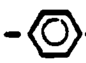 $-Cl$ | 1 | $-SCH_3$ |
| $-CH_2-$ 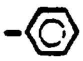 $-OCF_3$ | 1 | $-SCH_3$ |
| $-CH_2-$ 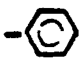 $-CF_3$ | 1 | $-SCH_3$ |
| H | 1 | $-S-CH_2-$ 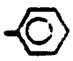 |
| $-C_4H_9-n$ | 1 | $-S-CH_2-$ 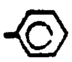 |
| $-CH_2-$ 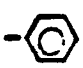 $-Cl$ | 1 | $-S-CH_2-$ 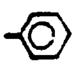 |
| $-CH_2-$ 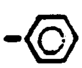 $-OCF_3$ | 1 | $-S-CH_2-$ 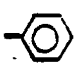 |
| $-CH_2-$ 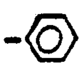 $-CF_3$ | 1 | $-S-CH_2-$ 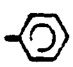 |
| H | 1 | $-S-CH_2-$ 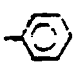 $-Cl$ |
| $-C_4H_9-n$ | 1 | $-S-CH_2-$ 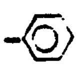 $-Cl$ |
| $-CH_2-$ 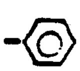 $-Cl$ | 1 | $-S-CH_2-$ 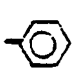 $-Cl$ |
| $-CH_2-$ 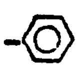 $-OCF_3$ | 1 | $-S-CH_2-$ 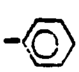 $-Cl$ |
| $-CH_2-$ 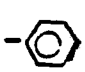 $-CF_3$ | 1 | $-S-CH_2-$ 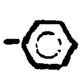 $-Cl$ |

| $R^1$ | n | $R^2$ |
|---|---|---|
| H | 1 | 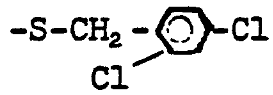 |
| $-C_4H_9-n$ | 1 | 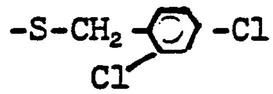 |
| 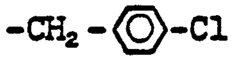 | 1 | 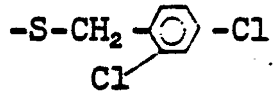 |
| 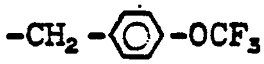 | 1 | 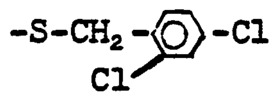 |
| 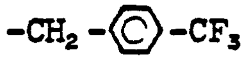 | 1 | 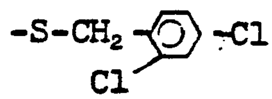 |
| H | 1 | 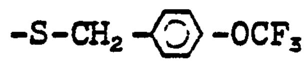 |
| $-C_4H_9-n$ | 1 | 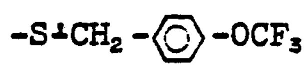 |
| 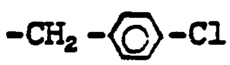 | 1 | 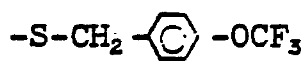 |
| 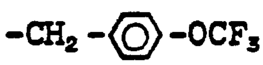 | 1 | 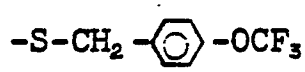 |
| 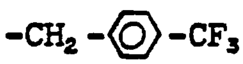 | 1 | 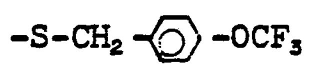 |
| H | 1 | 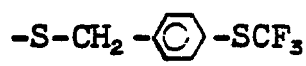 |
| $-C_4H_9-n$ | 1 | 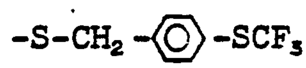 |
| 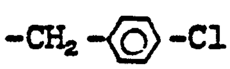 | 1 | 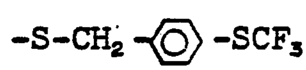 |
| 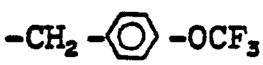 | 1 | 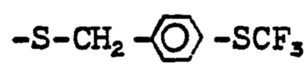 |
| 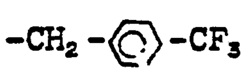 | 1 | 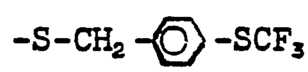 |

| $R^1$ | n | $R^2$ |
|---|---|---|
| H | 1 | 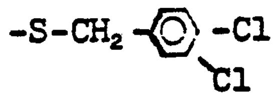 |
| $-C_4H_9-n$ | 1 | 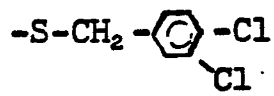 |
| 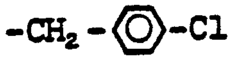 | 1 | 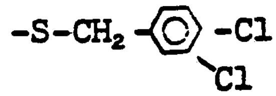 |
| 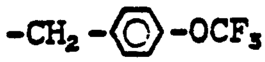 | 1 | 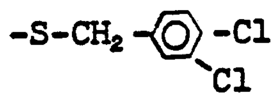 |
| 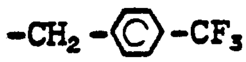 | 1 | 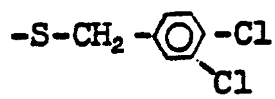 |
| H | 1 | 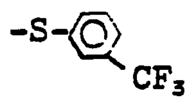 |
| $-C_4H_9-n$ | 1 | 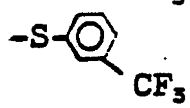 |
| 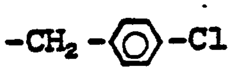 | 1 | 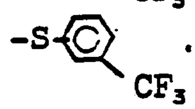 |
| 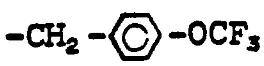 | 1 | 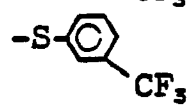 |
| 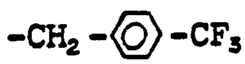 | 1 | 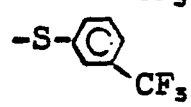 |
| H | 1 | 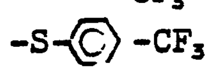 |
| $-C_4H_9-n$ | 1 | 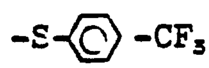 |
| 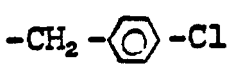 | 1 | 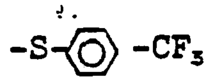 |
| 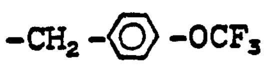 | 1 | 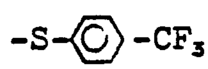 |
| 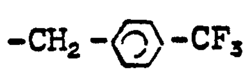 | 1 | 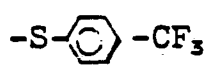 |

| $R^1$ | n | $R^2$ |
|---|---|---|
| H | 1 | 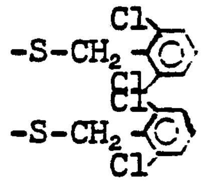 |
| $-C_4H_9-n$ | 1 | |
| 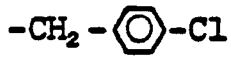 | 1 | 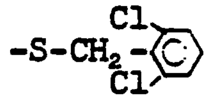 |
| 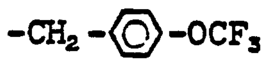 | 1 | 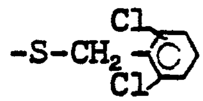 |
| 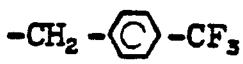 | 1 | 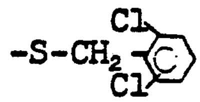 |
| H | 1 | 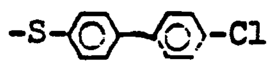 |
| $-C_4H_9-n$ | 1 | 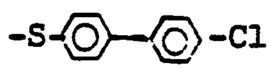 |
| 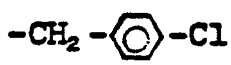 | 1 | 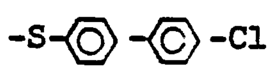 |
| 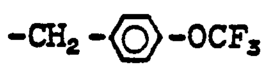 | 1 | 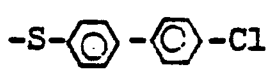 |
| 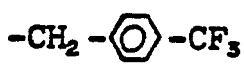 | 1 | 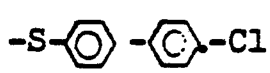 |
| H | 1 | 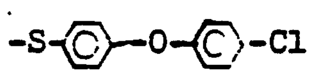 |
| $-C_4H_9-n$ | 1 | 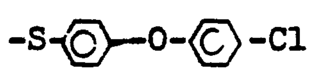 |
| 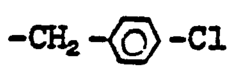 | 1 | 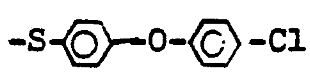 |
| 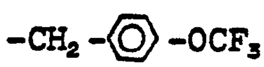 | 1 | 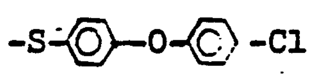 |
| 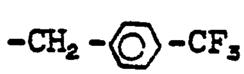 | 1 | 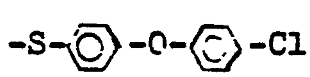 |

| $R^1$ | n | $R^2$ |
|---|---|---|
| H | 1 | 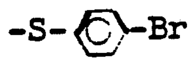 |
| $-C_4H_9$-n | 1 | 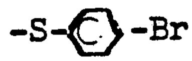 |
| 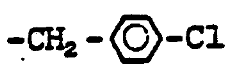 | 1 | 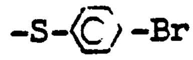 |
| 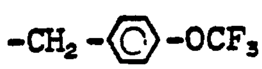 | 1 | 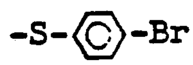 |
| 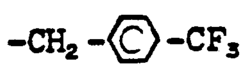 | 1 | 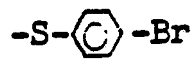 |
| H | 1 | 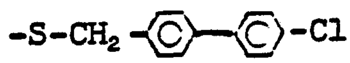 |
| $-C_4H_9$-n | 1 | 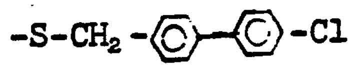 |
| 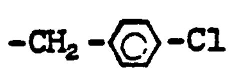 | 1 | 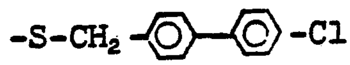 |
| 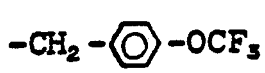 | 1 | 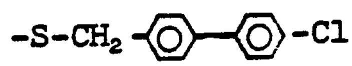 |
| 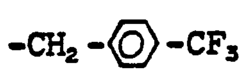 | 1 | 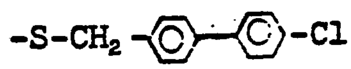 |
| H | 1 | 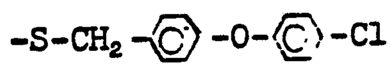 |
| $-C_4H_9$-n | 1 | 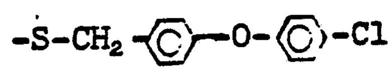 |
| 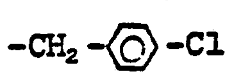 | 1 | 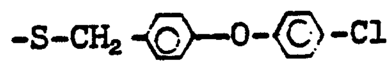 |
| 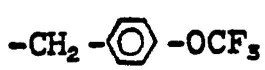 | 1 | 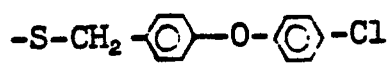 |
| 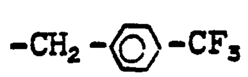 | 1 | 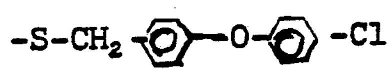 |

| $R^1$ | n | $R^2$ |
|---|---|---|
| H | 1 | 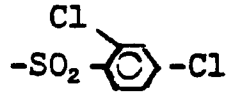 |
| $-C_4H_9-n$ | 1 | 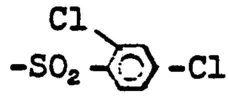 |
| 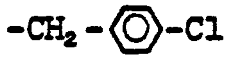 | 1 | 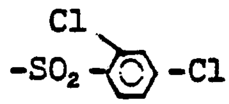 |
| 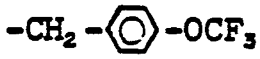 | 1 | 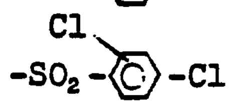 |
| 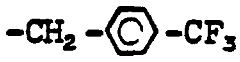 | 1 | 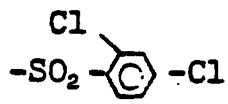 |
| H | 1 | 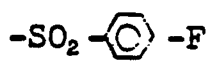 |
| $-C_4H_9-n$ | 1 | 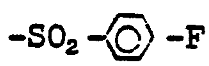 |
| 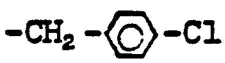 | 1 | 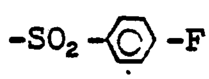 |
| 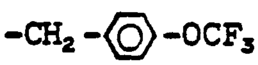 | 1 | 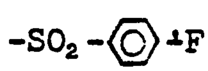 |
| 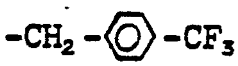 | 1 | 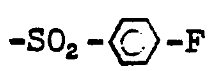 |
| H | 1 | 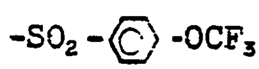 |
| $-C_4H_9-n$ | 1 | 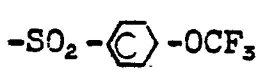 |
| 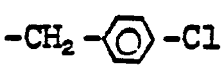 | 1 | 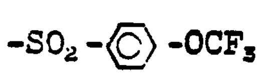 |
| 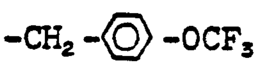 | 1 | 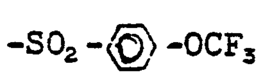 |
| 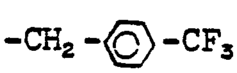 | 1 | 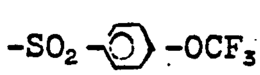 |

| $R^1$ | n | $R^2$ |
|---|---|---|
| H | 1 | $-SO_2-CH_2-$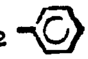$-Cl$ |
| $-C_4H_9-n$ | 1 | $-SO_2-CH_2-$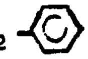$-Cl$ |
| $-CH_2-$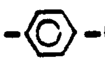$-Cl$ | 1 | $-SO_2-CH_2-$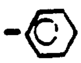$-Cl$ |
| $-CH_2-$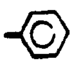$-OCF_3$ | 1 | $-SO_2-CH_2-$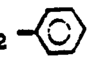$-Cl$ |
| $-CH_2-$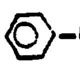$-CF_3$ | 1 | $-SO_2-CH_2-$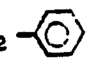$-Cl$ |

Verwendet man beispielsweise 1-Brom-3-(4-chlorphenoxy)-3-methyl-butan-2-on und 1,2,4-Triazol als Ausgangsstoffe, so kann der Reaktionsablauf durch das folgende Formelschema wiedergegeben werden (Verfahren a):

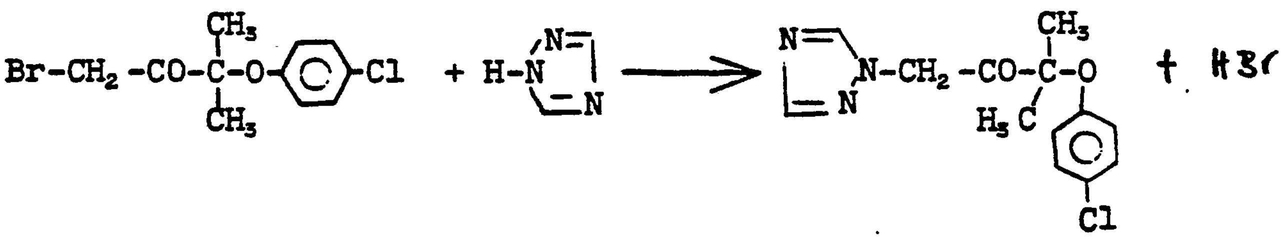

Verwendet man beispielsweise 3-(2,4-Dichlorphenoxy)-3-methyl-1-(1,2,4-triazol-lyl)-butan-2-on und 4-Chlorbenzylchlorid als Ausgangsstoffe, so kann der Reaktionsablauf durch das folgende Formelschema wiedergegeben werden (Verfahren b):

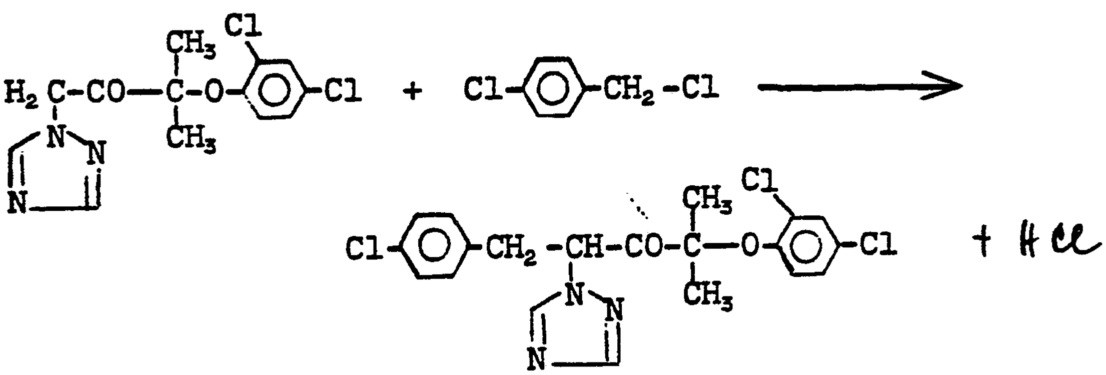

Verwendet man beispielsweise 4-(4-Chlorphenylthio)-3,3-dimthyl-1-(imidazol-1-yl)-butan-2-on und Wasserstoffperoxid in Eisessig als Ausgangsstoffe, so kann der Reaktionsablauf durch das folgende Formelschema wiedergegeben werden (Verfahren c):

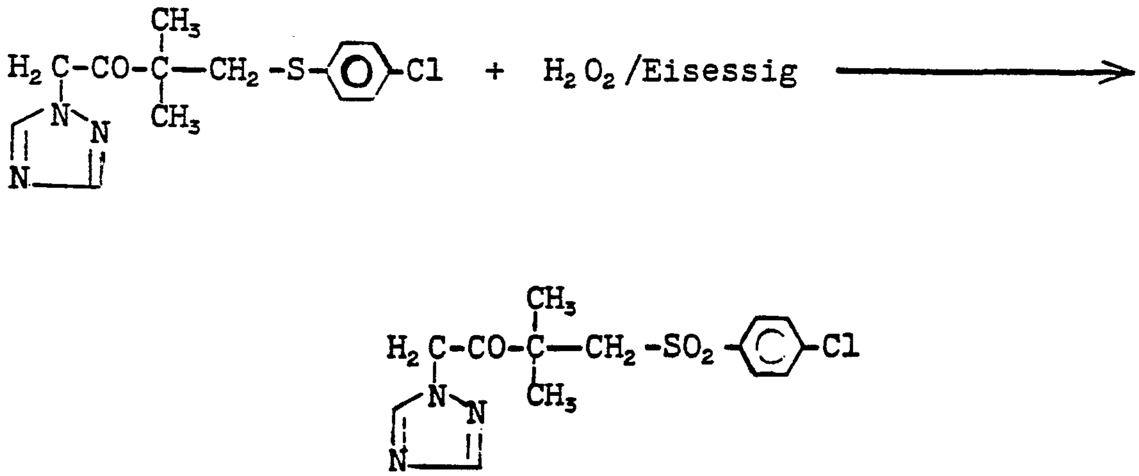


Die für die Durchführung des erfindungsgemäßen Verfahrens (a) als Ausgangsstoffe benötigten Halogenketone sind durch die Formel (II) allgemein definiert. In dieser Formel stehen $\underline{R}^2$ und der Index $\underline{n}$ für die in der Erfindungsdefinition angegebenen Bedeutungen.

Die Halogenketone der Formel (II) sind teilweise bekannt (vergleiche DE-OS 26 35 663), teilweise sind sie Gegenstand eigener älterer Anmeldungen, die noch nicht veröffentlicht sind (vergleiche z.B.deutsche Patentanmeldung P 30 21 551 vom 7.6.1980 und teilweise sind sie völlig neu. Sie werden erhalten, indem man Ketone der Formel

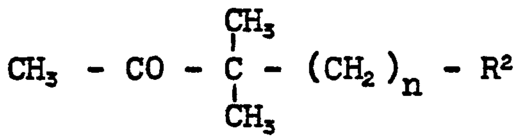
(V)

in welcher
$R^2$ und n die oben angegebene Bedeutung haben,
mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei 20 bis 60°C umsetzt.

Die Ketone der Formel (V) sind teilweise bekannt [vergleiche J.Org.Chem.$\underline{42}$, 1709-1717(1977); J.Am.Chem.Soc. $\underline{98}$, 7882-84 (1976); J.Org.Chem. $\underline{37}$, 2834-2840(1972), US-Patentschrift 3 937 738 sowie C.A. $\underline{82}$, 30 898 j (1975); teilweise sind sie Gegenstand eigener älterer Anmeldungen, die noch nicht veröffentlicht sind (vergleiche z.B. deutsche Patentanmeldung P 30 21 516 vom 7.6.1980; teilweise sind sie neu. Sie können nach den dort angegebenen Verfahren erhalten werden, indem man z.B. Keto-Derivate der Formel

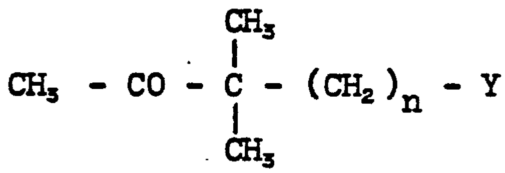
(VI)

in welcher
n die oben angegebene Bedeutung hat und
Y für Chlor, Brom oder die Gruppierung $-O-SO_2-R^6$ steht, wobei
$R^6$ für Alkyl mit 1 bis 4 Kohlenstoffatomen sowie für gegebenenfalls durch Alkyl mit 1 bis 4 Kohlenstoffatomen substituiertes Phenyl steht,
mit Verbindungen der Formel
Me - $R^2$ (VII)
in welcher
$R^2$ die oben angegebene Bedeutung hat und
Me für ein Alkalimetall, wie vorzugsweise Natrium und Kalium, oder Wasserstoff steht, in Gegenwart eines organischen Lösungsmittels, wie beispielsweise Xylol, Glykol oder Dimthylformamid, und gegebenenfalls in Gegenwart eines Säurebindemittels, wie beispielsweise Natriumcarbonat, bei Temperaturen zwischen 80 und 150°C umsetzt.

Die Keto-Derivate der Formel (VI) sind bekannt [vergleiche z.B. DE-08 26 32 603 und J.Org.Chem. $\underline{35}$, 2391

(1970)], bzw. können in allgemein bekannter Art und Weise erhalten werden.

Die Verbindungen der Formel (VII) sind allgemein bekannte Verbindungen der organischen Chemie und werden gegebenenfalls in-situ eingesetzt.

Die außerdem für das erfindungsgemäße Verfahren (a) als Ausgangsstoffe zu verwendenden Azole sind durch die Formel (III) allgemein definiert. In dieser Formel steht Az für 1,2,4-Triazol-1-yl und -4-yl sowie Imidazol-1-yl.

Die Azole der Formel (III) sind allgemein bekannte Verbindungen der organischen Chemie.

Die für die Durchführung des erfindungsgemäßen Verfahrens (b) als Ausgangsstoffe zu verwendenden Verbindungen sind durch die Formel (Ia) allgemein definiert. Die Verbindungen der Formel (Ia) sind erfindungsgemäße Stoffe.

Die außerdem für das erfindungsgemäße Verfahren (b) als Ausgangsstoffe zu verwendenden Alkylierungsmittel sind durch die Formel (IV) allgemein definiert. In dieser Formel steht $R^1$ vorzugsweise für diejenigen Reste, die bereits im Zusammenhang mit der Beschreibung der erfin-dungsgemäßen Stoffe der Formel (I) vorzugsweise für diesen Substituenten genannt wurden. Z steht vorzugsweise für eine elektronenanziehende Abgangsgruppierung, wie beispielsweise Halogen, p-Methylphenylsulfonyloxy, die Gruppierung $-0-80_2-OR$ oder $NR_3$.

Die Alkylierungsmittel der Formel (IV) sind allgemein bekannte Verbindungen der organischen Chemie.

Die für die Durchführung des erfindungsgemäßen Verfahrens (c) als Ausgangsstoffe zu verwendenden Verbindungen sind durch die Formel (Ib) allgemein definiert. Die Verbindungen der Formel (Ib) sind erfindungsgemäße Stoffe.

Die erfindungsgemäße Oxidation erfolgt durch die Umsetzung mit üblichen anorganischen oder organischen Oxidationsmitteln. Hierzu gehören vorzugsweise organische Persäuren, wie z.B. Peressigsäure, p-Nitroperbenzoesäure, m-Chlorperbenzoesäure; anorganische Persäuren, wie z.B. Perjodsäure; weiterhin Wasserstoffperoxid in Eisessig oder Methanol, Kaliumpermanganat und Chromsäure.

Für das erfindungsgemäße Verfahren (a) kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol, oder Chlorbenzol; Formamide, wie insbesondere Dimethylformamid; und halogenierte Kohlenwasserstoffe.

Das erfindungsgemäße Verfahren (a) wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden Ueber-schuß an Azol.

Die Reaktionstemperaturen können bei dem erfindungsgemäßen Verfahren (a) in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) setzt man auf 1 Mol der Verbindungen der Formel (II) vorzugsweise 2 bis 4 Mol Azol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Für das erfindungsgemäße Verfahren (b) kommen als Verdünnungsmittel inerte organische Lösungsmittel infrage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Das erfindungsgemäße Verfahren (b) wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens (b) in einem größeren Bereich variiert werden. Im allgemeinen arbeitet man zwischen 0 und 100°C, vorzugsweise zwischen 20 und 100°C.

Bei der Durdhführung des erfindungsgemäßen Verfahrens (b) setzt man vorzugsweise auf 1 Mol der Verbindung der Formel (Ia) 1 bis 1,2 Mol Alkylierungsmittel ein. Die Isolierung der Endprodukte der Formel (I) erfolgt in allgemein üblicher Art und Weise.

Das erfindungsgemäße Verfahren (b) kann auch in einem Zweiphasensystem, wie beispielsweise wässrige Natronoder Kalilauge/Toluol oder Methylenchlorid, gegebenenfalls unter Zusatz von 0,1 bis 1 Mol eines Phasen-Transfer-Katalysators, wie beispielsweise Ammonium- oder phosphoniumverbindungen, Benzyldodecyldimethylammoniumchlorid oder Triethyl-benzyl-ammoniumchlorid, durchgeführt werden.

Die Reaktionstemperaturen können bei der Durchführung der Oxidation gemäß Verfahren (c) in einem größeren Bereich variiert werden. Im allgemeinen arbeitet man zwischen etwa -50 bis 100°C, vorzugsweise zwischen -30 und 80°C.

Bei der Durchführung der erfindungsgemäßen Oxidation gemäß Verfahren (c) setzt man auf 1 Mol der erfindungsgemäßen Verbindungen der Formel (Ib) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen -30 bis + 30°C, entstehen vorzugsweise die erfindungsgemäßen

Verbindungen der Formel (I) mit X = SO. Bei Ueberschuß an Oxidationsmittel und höheren Temperaturen (10 bis 80°C) entstehen vorzugsweise die erfindungsgemäßen Verbindungen der Formel (I) mit X = $SO_2$. Die Isolierung der Oxidationsprodukte erfolgt in üblicher Weise.

Die erfindungsgemäß herstellbaren Verbindungen der Formel (I) können in Säureadditionssalze bzw. Metallsalz-Komplexe überführt werden.

Zur Herstellung von physiologisch verträglichen Säure-additionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure. Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I und II. sowie IV. bis VIII. Nebengruppe infrage wobei Kupfer Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute pflanzenverträglichkeit der Wirkstoffe in den zur Bekämpfung von Pflanzenkrankheiten notwendigen Konzentrationen erlaubt eine Behandlung von oberirdischen pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gerste- bzw. des Getreidemehltaus (Erysiphe graminis), oder zur Bekämpfung von Podosphaera-Arten, wie z.B. gegen den Erreger des Apfelmehltaus (Podosphaera leucotricha). Hervorzuheben ist die teilweise systemische Wirkung der erfindungsgemäßen Stoffe. So gelingt es, Pflanzen gegen Pilzbefall zu schützen, wenn man den Wirkstoff über den Boden und die Wurzel den oberirdischen Teilen der Pflanze zuführt.

In entsprechenden Aufwandmengen zeigen die erfindungs-gemäßen Stoffe auch wachstumsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsatzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark Polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid: als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als

Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo -Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche,in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Herstellungsbeispiele**

**Beispiel 1**

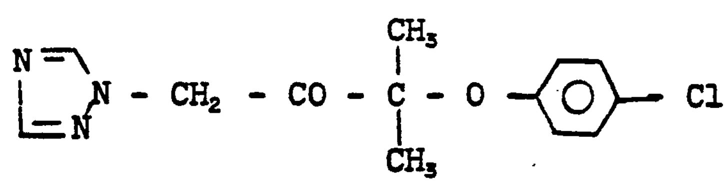

(Verfahren a)

In die siedende Mischung von 14g (0,2 Mol) Triazol und 14g (0,1 Mol) Kaliumcarbonat in 200 ml Aceton tropft man 20g (0,1 Mol) 1-Brom-3-(4-chlorphenoxy)-3-methyl-butan-2-on und läßt 5 Stunden unter Rückfluß nachreagieren. Anschließend wird der anorganische Rückstand abfiltriert, das Lösungsmittel im Wasserstrahlvakuum abdestilliert, der organische Rückstand in 200 ml Methylenchlorid/200 ml Wasser aufgenommen, die organische Phase abgetrennt, zweimal mit je 100 ml Wasser gewaschen, über Natriumsulfat getrocknet und das Lösungsmittel im Wasserstrahlvakuum abdestilliert. Der Rückstand wird aus Cyclohexan/Isopropanol (˜ 20: 1) umkristallisiert. Man erhält 17g (70 % der Theorie) 3-(4-Chlorphenoxy)-3-methyl-1-(1,2,4-triazol-1-yl)-butan-2-on vom Schmelzpunkt 101-103°C.

**Herstellung des Auagangsproduktes**

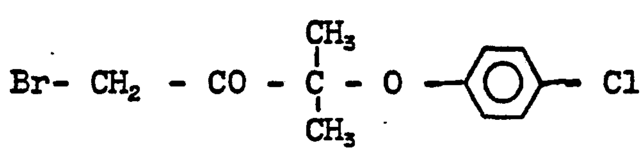

110,2g (0,5 Mol) 3-(4-Chlorphenoxy)-3-methyl-butan-2-on werden in 500 ml Methylenchlorid gelöst und 83 g (0,52 Mol) Brom bei Raumtemperatur so zugetropft, daß laufend Entfärbung eintritt. Nach beendeter Zugabe wird 30 Minuten nachgerührt, die organische Phase je zweimal mit 300 ml Wasser und 300 ml gesättigter

Natriumhydrogencarbonatlösung gewaschen, die organische Phase über Natriumsulfat getrocknet und destilliert. Man erhält 105,9 g (70 % der Theorie) 1-Brom-3-(4-chlorphenoxy)-3-methyl-butan-2-on vom Siedepunkt 115-118°C /0,1 Torr.

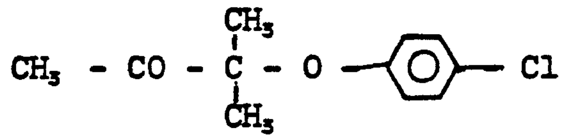

130 g (0,5 Mol) 3-Brom:3-methyl-butan-2-on, 120 g (0,9 Mol) p-Chlorphenol und 139g (1,0 Mol) Kaliumcarbonat werden in 500 ml Aceton unter Rühren 6 Stunden unter Rückfluß erhitzt. Man saugt den anorganischen Niederschlag ab, destilliert das Lösungsmittel im Wasserstrahlvakuum bei 20-30°C ab, nimmt den Rückstand in 300 ml Methylenchlorid/300 ml Wasser auf, trennt die wässrige Phase ab, wäscht die organische Phase zweimal mit je 100 ml 5-%iger Natronlauge, wäscht zweimal mit je 100 ml Wasser nach, trocknet die organische Phase über Natriumsulfat und destilliert. Man erhält 110,2 g (65 % der Theorie) 3-(4-Chlorphenoxy)-3-methyl-butan-2-on vom Siedepunkt 80-95°C/ 0,1 Torr und vom Brechungsindex
$n_D^{20}$= 1,5150.

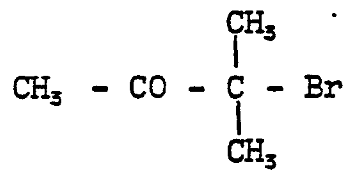

86 g (1 Mol) Methyl-isopropylketon werden in 500 ml Methylenchlorid gelöst und bei Raumtemperatur 159.8 g (1 Mol) Brom so zugetropft, daß laufend Entfärbung eintritt. Nach beendeter Zugabe läßt man 30 Minuten nachrühren. Die organische Lösung wird je zweimal mit 500 ml Wasser und 500 ml gesättigter Natriumhydrogencarbonatlösung gewaschen, über Natriumsulfat getrocknet und destilliert. Man erhält 130 g (80 % der Theorie) 3-Brom-3-methyl-butan-2-on vom Siedepunkt 39°C/12 Torr und vom Brechungsindex
$n_D^{20}$ = 1,4543.

**Beispiel 2**

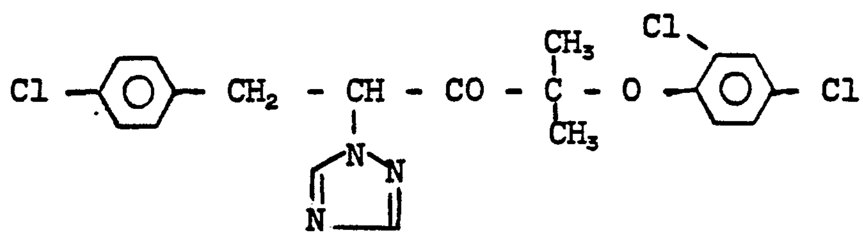

(Verfahren b)

Zu 31,4g (01 Mol) 3-(2,4-Dichlorphenoxy)-3-methyl-1-(1,2,4-triazol-1-yl)-butan-2-on (Herstellung entsprechend Beispiel l)in 100 ml Dimethylsulfoxid werden unter Kühlung bei 20°C zunächst 5,6g Kaliumhydroxid in 12 ml Wasser und danach 16,1g (0,1 Mol) 4-Chlorbenzylchlorid in 5 ml Dimethylsulfoxid getropft. Man läßt 15 Stunden bei 20°C nachreagieren, gibt die Lösung auf 200 ml Wasser, extrahiert mit 200 ml Methylenchlorid, wäscht die organische Phase dreimal mit je 200 ml Wasser, trocknet über Natriumsulfat und destilliert das Lösungsmittel im Wasserstrahlvakuum ab. Der Rückstand wird in 200 ml Ether aufgenommen, unter Rückfluß erhitzt und die ausgefallenen Kristalle abgesaugt.

Man erhält 21,3g (48 % der Theorie) 5-(4-Chlorphenyl)-2-(2,4-dichlorphenoxy)-2-methyl-4-(1,2,4-triazol-1-yl)-pentan-3-on vom Schmelzpunkt 104-108°C.

**Beispiel 3**

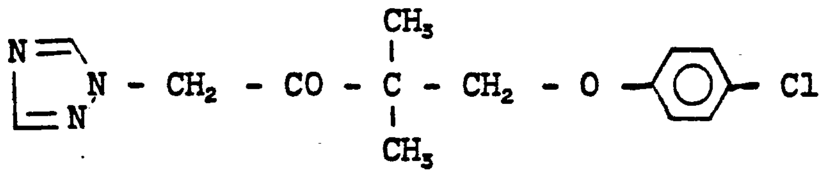

(Verfahren a)

Zu 200g (2,9 Mol) Triazol und 140 g (1 Mol) Kaliumcarbonat in 1000 ml Aceton werden in der Siedehitze 304 g (1 Mol) 4-Brom-1-(4-chlorphenoxy)-2,2-dimethyl-butan-3-on getropft. Man läßt 15 Stunden unter Rück-fluß nachreagieren, saugt den anorganischen Niederschlag ab, destilliert das Lösungsmittel im Wasserstrahlvakuum ab, nimmt den Rückstand in 2 1 Methylenchlorid auf und wäscht die organische Phase viermal mit je 500 ml Wasser, trocknet über Natriumsulfat und engt durch Abdestillieren des Lösungsmittels im Vakuum ein. Der Rückstand wird in 500 ml Ether aufgenommen, wobei er auskristallisiert. Man erhält 201,8g (69 % der Theorie) 1-(4-Chlorphenoxy)-2,2-dimethyl-4-(1,2,4-triazol-1-yl)-butan-3-on vom Schmelzpunkt 90-92° C.

**Herstellung des Ausgangsproduktes**

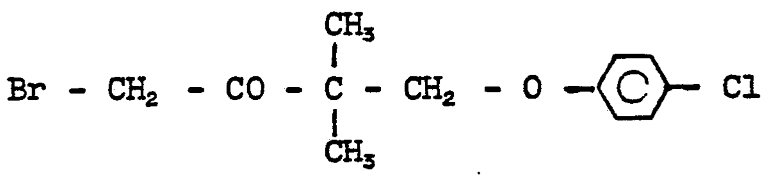

36 g (0,159 Mol) 1-(4-Chlorphenoxy)-2,2-dimethyl-butan-3-on werden in 300 ml Chloroform gelöst und bei 20° C tropfenweise so mit 25,5 g (0,159 Mol) Brom versetzt, daß laufend Entfärbung eintritt. Nach beendeter Zugabe läßt man 30 Minuten bei Raumtemperatur rühren und engt danach durch Abdestillieren des Lösungsmittels im Vakuum ein. Man erhält 48,5 g (quantitativer Umsatz) rohes 1-Brom-4-(4-chlorphenoxy)-3,3-dimethyl-butan-2-on als Oel.

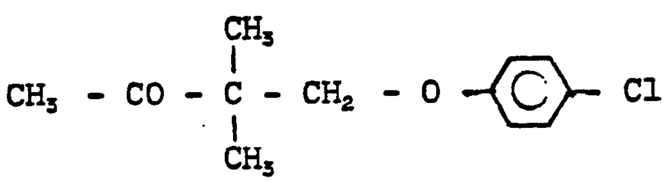

29,7g (0,55 Mol) Natriummethylat werden in 500 ml Methanol gelöst und unter Rühren mit 70,4g (0,55 Mol) 4-Chlorphenol versetzt. Nach 10 Minuten Rühren wird das Lösungsmittel unter vermindertem Druck abdestilliert und der Rückstand in 100 ml Glykol aufgenommen. Diese Lösung wird zu einer Lösung von 135 g (0,5 Mol) 2,2-Dimethyl-1-tosyloxy-butan-3-on in 200 ml Glykol gegeben. Man läßt 48 Stunden bei 100 bis 120° C rühren, kühlt an und rührt das Reaktionsgemisch in 2000 ml Wasser ein. Man extrahiert zweimal mit je 250 ml Diethylether, wäscht die vereinigten organischen Phasen dreimal mit je 100 ml Wasser, einmal mit 100 ml 10%-iger Natronlauge und nochmals mit 100 ml Wasser, trocknet über Natriumsulfat und destilliert.

Man erhält 62,9 g (55,7 % der Theorie) 1-(4-Chlorphen-oxy)-2,2-dimethyl-butan-3-on vom Siedepunkt 135-140° C/ 0,4 Torr.

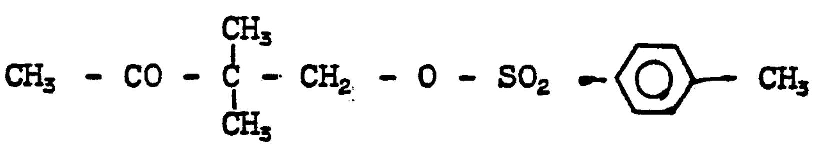

47,6 g (0,25 Mol) 4-Toluolsulfochlorid werden in 100 ml Chloroform gelöst, 35g (0,3 Mol) 2,2-Dimethyl-1-hydroxy-butan-3-on zugegeben und bei 0 bis 5° C 40 ml (0,5 Mol) Pyridin zugetropft. Man läßt 15 Stunden bei Raumtemperatur nachrühren, gibt das Reaktionsgemisch auf 200 g Eis und 70 ml konzentrierter Salzsäure, trennt die organische Phase ab, wäscht sie dreimal mit je 200 ml Wasser nach, trocknet über Natriumsulfat und engt ein. Der Rückstand wird in 100 ml Petrolether aufgenommen, wobei das Endprodukt auskristallisiert. Man erhält 46 g (71 % der Theorie) 2,2-Dimethyl-1-tosyloxy-butan-3-on als farblose Kristalle vom Schmelzpunkt 49-52° C.

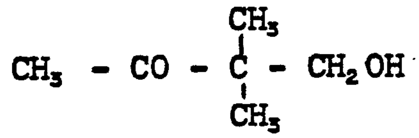

Zu 172 g (2 Mol) Methylisopropylketon in 1000 ml Methanol werden 66 g (2,2.Mo1) Paraformaldehyd und 1 g Kaliumhydroxid in 10 ml Methanol getropft. Man erhitzt 15 Stunden unter Rückfluß und destilliert anschließend das Methanol über eine Kolonne bei 82°C Innentemperatur ab. Der Rückstand wird im Wasserstrahlvakuum destilliert. Man erhält 152,7g (68 % der Theorie) 2,2-Dimethyl-1-hydroxy-butan-3-on vom Siedepunkt 80-82°C/12 Torr.

**Beispiel 4**

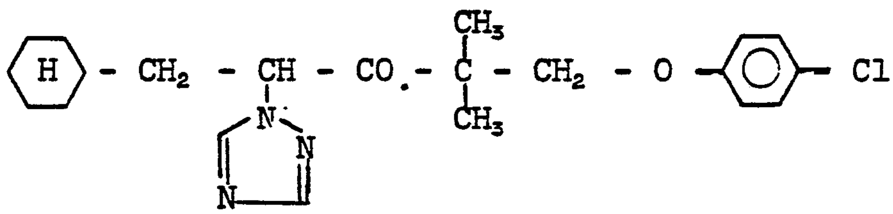

(Verfahren b)

Zu 29,3 g (0,1 Mol) 1-(4-Chlorphenoxy)-2,2-dimethyl-4-(1,2 4-triazol-1-yl)-butan-3-on (Beispiel 3) in 100 ml Dimethylsulfoxid werden bei 20°C zunächst 5,6 g Kaliumhydroxid in 12 ml Wasser und danach 17,7 g (0,1 Mol) Cyclohexylmethylbromid in 5 ml Dimethylsulfoxid getropft. Man läßt das Reaktionsgemisch 15 Stunden bei Raumtemperatur nachrühren, gibt es auf 200 ml Wasser und extrahiert mit 200 ml Methylenchlorid. Die organische Phase wird dreimal mit je 100 ml Wasser gewaschen, über Natriumsulfat getrocknet und durch Abdestillieren des Lösungsmittels eingeengt. Der Rückstand wird in 100 ml Ether aufgenommen, wobei er auskristallisiert. Man erhält 18,6 g (47 % der Theorie) 1-(4-Chlorphenoxy)-5-cyclohexyl-2,2-dimethyl-4-(1,2,4-triazol-1-yl)-pentan-3-on vom Schmelzpunkt 58-60°C.

**Beispiel 5**

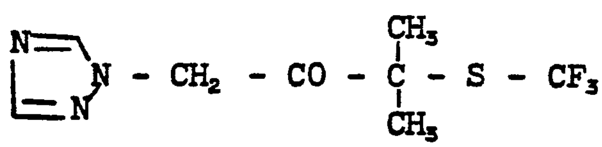

(Verfahren a)

Zu einer Mischung von 8,3 g (0,12 Mol) 1,2,4-Triazol und 28 g (0,2 Mol) Kaliumcarbonat in 150 ml Aceton tropft man bei Raumtemperatur 26,5 g (0,1 Mol) 1-Brom-3-methyl-3-trifluormethylthio-butan-2-on. Man läßt 1 Stunde unter Rückfluß nachrühren, saugt die anorganischen Salze ab und engt das Filtrat ein. Der Rückstand wird in Methylenchlorid aufgenommen, mit Wasser gewaschen, über Natriumsulfat getrocknet und destilliert. Man erhält 15,5g (62 % der Theorie) 3-Methyl-1-(1,2,4-triazol-1-yl)-3-trifluormethylmercapto-butan-2-on vom Siedepunkt 91 °C/0,5 Torr und vom Schmelzpunkt ~45°C.

**Beispiel 6**

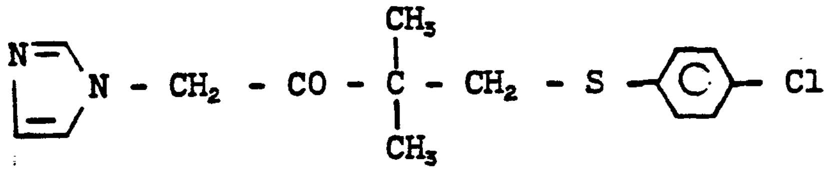

(Verfahren a)

199 g (0,618 Mol) 1-Brom-4-(4-chlorphenylmercapto)-3,3-dimethyl-butan-2-on, 120 g (1,76 Mol) Imidazol und 243,5 g (1,76 Mol) Kaliumcarbonat in 3 1 Aceton werden 5 Stunden unter Rückfluß gerührt. Danach läßt man abkühlen, saugt die anorganischen Salze ab und engt das Filtrat ein. Der Rückstand wird in Methylenchlorid aufgenommen, dreimal mit Wasser gewaschen, über Natriumsulfat getrocknet und eingeengt. Nach Umkristallisation aus Diisopropylether erhält man 156 g (82 % der Theorie) 4-(4-Chlorphenylmercapto)-3,3-dimethyl-1-(imidazol-1-yl)-butan-2-on vom Schmelzpunkt 50°C.

**Herstellung der Ausgangsprodukte**

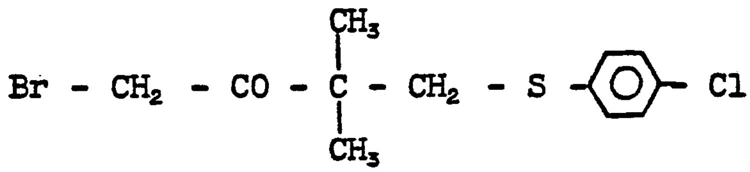

97 g (0,4 Mol) 1-(4-Chlorphenylmercapto)-2,2-dimethyl-butan-3-on werden bei Raumtemperatur langsam mit 64 g (0,4 Mol) Brom versetzt. Das Reaktionsgemisch wird entsprechend Beispiel 1 aufgearbeitet. Man erhält 127 g (99 % der Theorie) 1-Brom-4-(4-chlorphenylmercapto)-3,3-dimethyl-butan-2-on als zähflüssiges Oel.

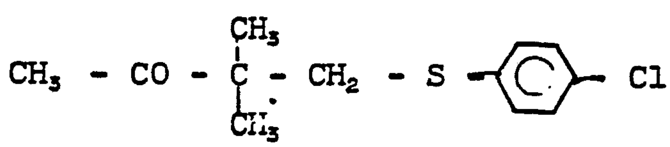

134,5 g (1 Mol) 4-Chlorpinakolin werden mit 216 g (1,5 Mol) 4-Chlorthiophenol und 210g (1,52 Mol) Kaliumcarbonat in 500 ml Dimethylformamid 15 Stunden bei 150°C und einem Druck von 2 bis 4 bar gerührt. Man läßt auf Raumtamperatur abkühlen, verrührt mit 10 1 Wasser und extrahiert mit Ether. Die Etherphase wird über Natriumsulfat getrocknet, eingeengt und der Rückstand im Vakuum destilliert. Man erhält 151g (62 % der Theorie) 1-(4-Chlorphenylmercapto)-2,2-dimethyl-butan-3-on vom Siedepunkt 146°C/0,5 Torr.

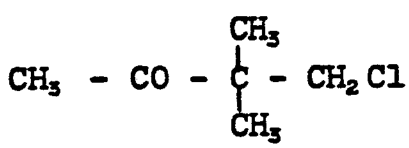

11,6 g (0,1 Mol) 2,2-Dimethyl-1-hydroxy-butan-3-on (Herstellung vgl.Beispiel 3) werden bei 50 bis 60°C (Eiskühlung) zu 20,5g (0,1 Mol) N,N- Diethyl-1,2,2-trichlor-vinyl-amin getropft. Nach zweistündigem Rühren bei 60°C wird im Wasserstrahlvakuum destilliert. Man erhält 8,1g (60 % der Theorie) 4-Chlorpinakolin vom Schmelzpunkt 60 - 62 °C/12 Torr. In entsprechender Weise und gemäß den angegebenen Verfahren werden die folgenden erfindungsgemäßen Verbindungen der allgemeinen Formel

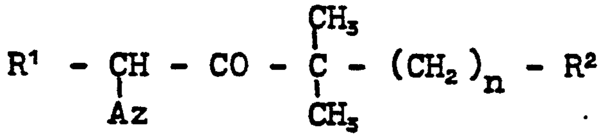 (I)

erhalten:

| Bsp. Nr. | $R^1$ | Az | n | $R^2$ | Schmelzpunkt (°C), Brechungsindex ($n_D^{20}$), Siedepunkt (°C/Torr) |
|---|---|---|---|---|---|
| 7 | H | 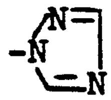 | 0 | 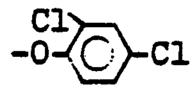 | 58 |
| 8 | H | 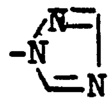 | 0 | 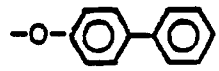 | 107 |
| 9 | H | 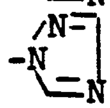 | 0 | 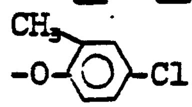 | 60-65 |
| 10 | H | 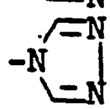 | 0 | 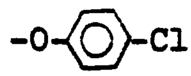 | 140 |
| 11 | 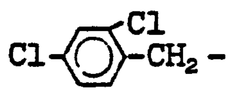 | 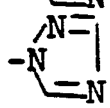 | 0 | 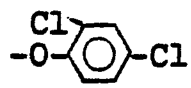 | 102 |
| 12 | 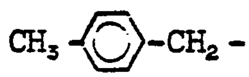 | 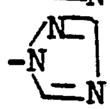 | 0 | 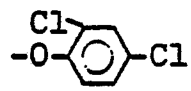 | 88-91 |
| 13 | 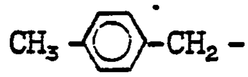 | 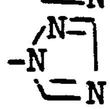 | 0 | 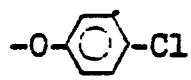 | 92-94 |
| 14 | 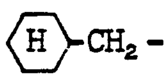 | 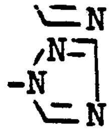 | 0 | 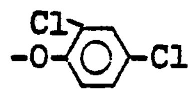 | 1,5490 |
| 15 | 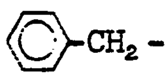 | 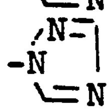 | 0 | 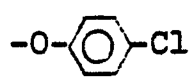 | 102-03 |
| 16 | 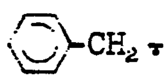 | 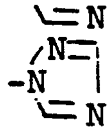 | 0 | 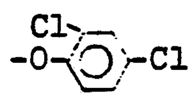 | 85-86 |
| 17 | 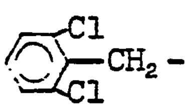 | 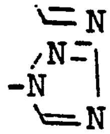 | 0 | 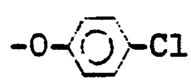 | 88-90 |

| Bsp. Nr. | $R^1$ | Az | n | $R^2$ | Physikal. Konstante |
|---|---|---|---|---|---|
| 18 | 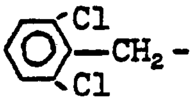 | 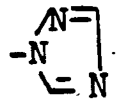 | 0 | 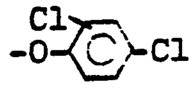 | 1,5831 |
| 19 | $C_4H_9$-n | 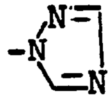 | 0 | 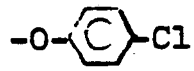 | 190-95/0,2 |
| 20 | $C_4H_9$ - n | 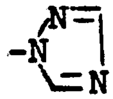 | 0 | 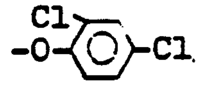 | 182-85/0,2 |
| 21 | 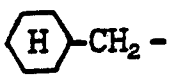 | 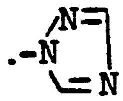 | 0 | 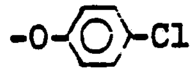 | 1,5380 |
| 22 | H | 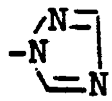 | 1 | $-O-CH_3$ | zähes Oel |
| 23 | H | 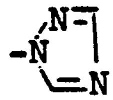 | 1 | 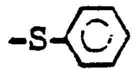 | 1,5720 |
| 24 | H | 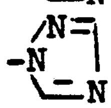 | 1 | 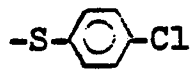 | o,5703 |
| 25 | H | 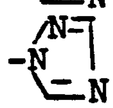 | 1 | 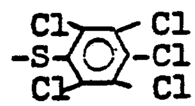 | 118-20 |
| 26 | H | 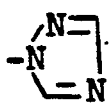 | 1 | 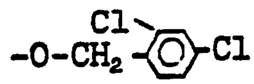 | 1,5428 |
| 27 | H | 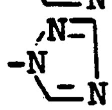 | 1 | 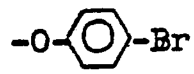 | zähes Oel |
| 28 | 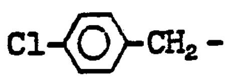 | 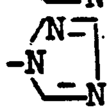 | 1 | 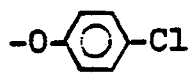 | 138-48 (xHCl) |
| 29 | 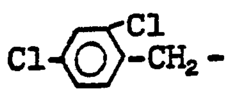 | 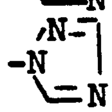 | 1 | $-OCH_3$ | 75-6 |
| 30 | 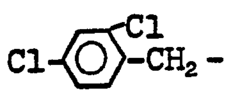 | 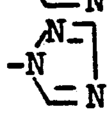 | 1 | 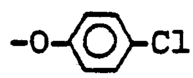 | 120 |
| 31 | 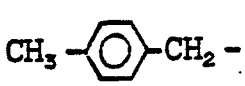 | 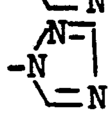 | 1 | 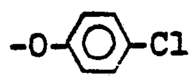 | 94 |
| 32 | 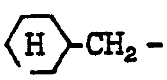 | 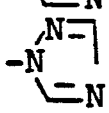 | 1 | $-OCH_3$ | 1,4788 |
| 33 | 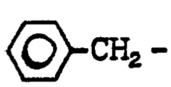 | 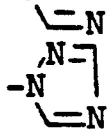 | 1 | 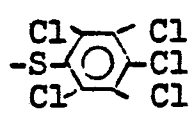 | 122-24 |

| Bsp. Nr. | $R^1$ | Az | n | $R^2$ | Physikal. Konstanten |
|---|---|---|---|---|---|
| 34 | H | 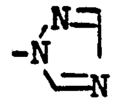 | 0 | $-SCF_3$ | 130(Zers.) (x $CuCl_2$) |
| 35 | H | 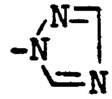 | 0 | 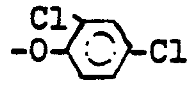 | 210(x$CuCl_2$) |
| 36 | H | 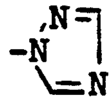 | 1 | 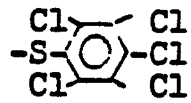 | 168(Zers.) (x$CuCl_2$) |
| 37 | H | 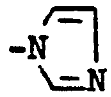 | 1 | 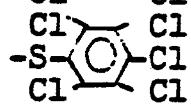 | 152(Zers.) |
| 38 | H | 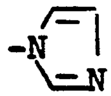 | 0 | 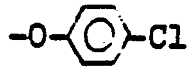 | zähes Oel |
| 39 | H | 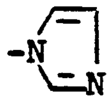 | 0 | 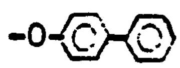 | 84-86 |
| 40 | H | 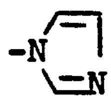 | 0 | 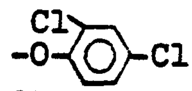 | zähes Oel |
| 41 | H | 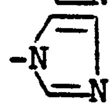 | 0 | 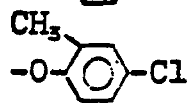 | zähes Oel |
| 42 | 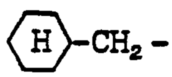 | 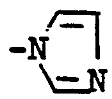 | 1 | 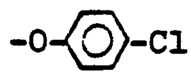 | 92-96 |
| 43 | H | 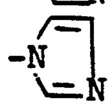 | 1 | 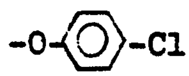 | 190(Zers.) (x HCl) |
| 44 | H | 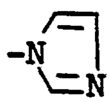 | 1 | 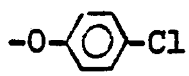 | zähes Oel |
| 45 | H | 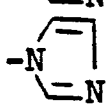 | 1 | 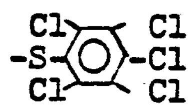 | 86-88 |
| 46 | H | 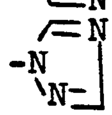 | 1 | $-OC_2H_5$ | 68-72 (xHCl) |
| 47 | H | 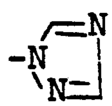 | 1 | 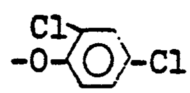 | 1,5392 |
| 48 | H | 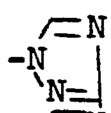 | 1 | 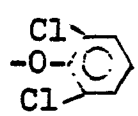 | 1,5428 |
| 49 | 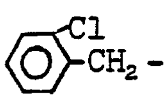 | 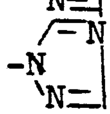 | 0 | 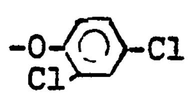 | 101-04 |

| Bsp. Nr. | $R^1$ | Az | n | $R^2$ | Physikal. Konstante |
|---|---|---|---|---|---|
| 50 | 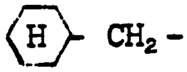 | 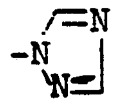 | 1 | 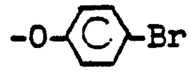 | 138-41° (xHCl) |
| 51 | 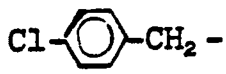 | 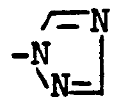 | 1 | 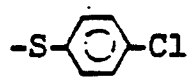 | 128-130 |
| 52 | 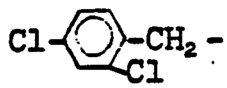 | 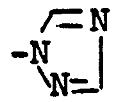 | 1 | 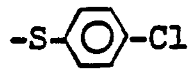 | 80 |
| 53 | $C_4H_9n$- | 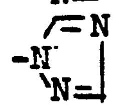 | 1 | 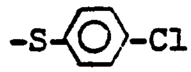 | 1,5563 |
| 54 | 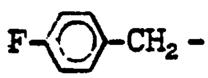 | 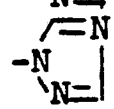 | 1 | 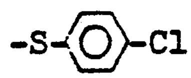 | 88-89 |
| 55 | 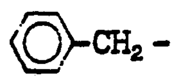 | 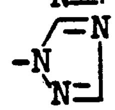 | 1 | 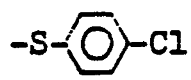 | 1,5806 |
| 56 | 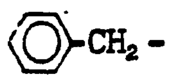 | 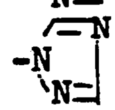 | 1 | 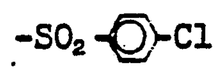 | 141-43 |
| 57 | 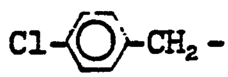 | 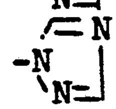 | 1 | $-OC_2H_5$ | 58-60 |
| 58 | 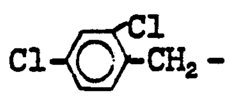 | 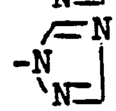 | 1 | 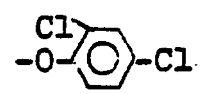 | 118-30 |
| 59 | H | 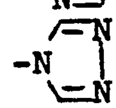 | 1 | 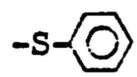 | 110 |
| 60 | 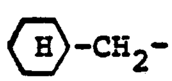 | 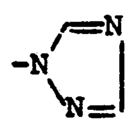 | 1 | 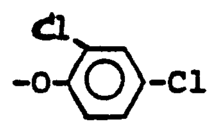 | 98-110 (xHCl) |
| 61 | H | 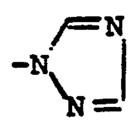 | 1 | 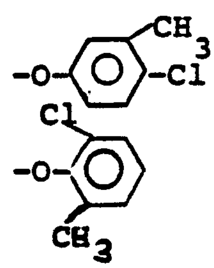 | 93-95 |
| 62 | H | 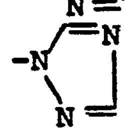 | 1 | | 170-75 (x HCl) |
| 63 | H | 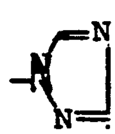 | 1 | 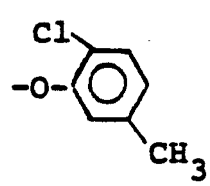 | 146-47 (xHCl) |

| Bsp. Nr. | $R^1$ | Az | n | $R^2$ | Schmelzpkt. (°C) $n_D^{20}$, Siedep. (°C/Torr) |
|---|---|---|---|---|---|
| 64 | $CH_2=CH-CH_2-$ | 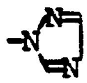 | 0 | 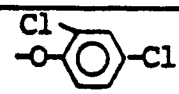 | 170-73 / 0,2 |
| 65 | $C_2H_5-$ | " | 0 | " | 172-75 / 0,2 |
| 66 | $CH_3-$ | " | 0 | " | 165-69 / 0,2 |
| 67 | $CH_2=CH-CH_2-$ | " | 0 | 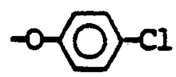 | 161-68 / 0,2 |
| 68 | $C_2H_5-$ | " | 0 | " | 165-70 / 0,25 |
| 69 | $CH_3-$ | " | 0 | " | 159-63 / 0,2 |
| 70 | $CH_3-$ | " | 0 | 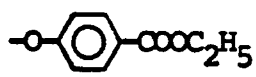 | 1,532 |
| 71 | $C_2H_5-$ | " | 0 | " | 185-90 / 0,1 |
| 72 | $CH_2=CH-CH_2-$ | " | 0 | " | 185-92 / 0,2 |
| 73 | $n-C_4H_9-$ | " | 0 | " | 195-200 / 0,2 |
| 74 | 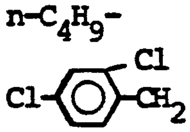 | " | 0 | " | 86-91 |
| 75 | 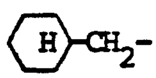 | " | 0 | " | 1,530 |
| 76 | 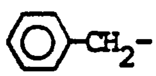 | 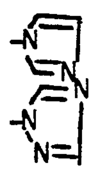 | 0 | " | 1,5660 |
| 77 | $CH_3-$ | | 1 | 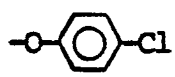 | 150 (xHCl) |
| 78 | $C_2H_5-$ | " | 1 | " | 160,5 (xHCl) |
| 79 | $n-C_4H_9-$ | " | 1 | $-OCH_3$ | 1,460 |
| 80 | H | " | 1 | 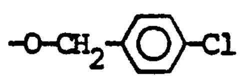 | 1,5298 |
| 81 | 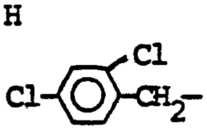 | " | 1 | 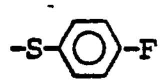 | 82 - 84 |
| 82 | 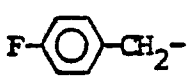 | " | 1 | 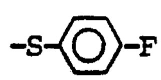 | Öl |
| 83 | H | " | 1 | 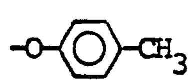 | 139 (xHCl) |
| 84 | H | " | 1 | " | 1,5263 |

| Bsp. Nr. | $R^1$ | Az | n | $R^2$ | Physikal. Konstante |
|---|---|---|---|---|---|
| 85 | H | 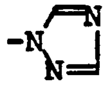 | 0 | 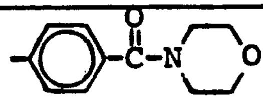 | 78-80 |
| 86 | $CH_2$=CH-$CH_2$ | " | 0 | 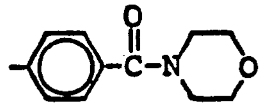 | $n_D^{20}$ = 1,5413 |
| 87 | (n)-$C_4H_9$- | " | 0 | 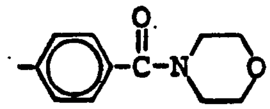 | $n_D^{20}$ = 1,5280 |
| 88 | 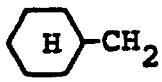 | " | 0 | 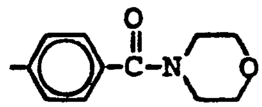 | $n_D^{20}$ = 1,5310 |
| 89 | 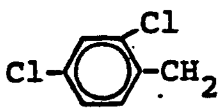 | 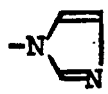 | 1 | 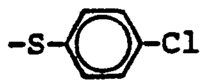 | Öl |
| 90 | 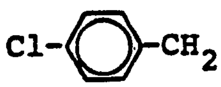 | " | 1 | " | Öl |
| 91 | 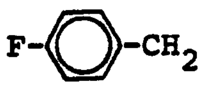 | " | 1 | " | Öl |
| 92 | (n)-$C_4H_9$- | " | 1 | " | Öl |
| 93 | $(CH_3)_2CH$- | " | 1 | " | Öl |
| 94 | 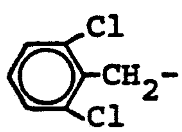 | 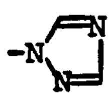 | 1 | " | 73 |
| 95 | 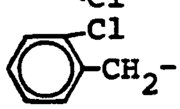 | " | 1 | " | 105 |
| 96 | H | " | 1 | 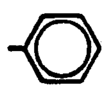 | 79 |
| 97 | H | 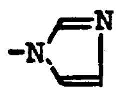 | 1 | 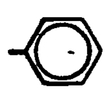 | 65 |

| Bsp. Nr. | $R^1$ | Az | n | $R^2$ | Physikal. Konstante |
|---|---|---|---|---|---|
| 98 | H | 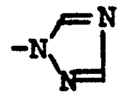 | 1 | 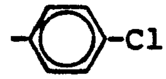 | 127 |
| 99 | H | " | 1 | 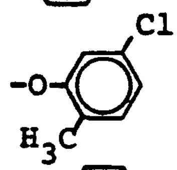 | 138 x HCl |
| 100 | H | 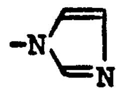 | 0 | 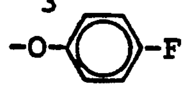 | 50-52 |
| 101 | H | 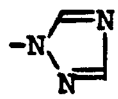 | 0 | " | 56-58 |
| 102 | 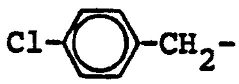 | " | 1 | 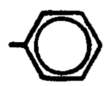 | 102 |
| 103 | 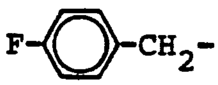 | " | 1 | $-OCH_3$ | Öl |
| 104 | 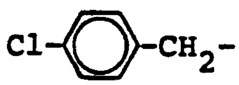 | " | 1 | $-OCH_3$ | Öl |
| 105 | (n)$-C_4H_9-$ | " | 1 | 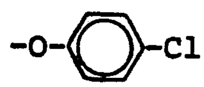 | Öl |
| 106 | $HC{\equiv}C-CH_2-$ | " | 1 | 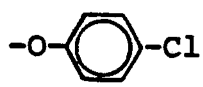 | Öl |
| 107 | 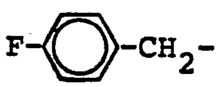 | " | 1 | 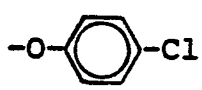 | Öl |
| 108 | $H_2C{=}CH-CH_2-CH_2-$ | " | 1 | 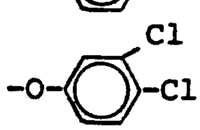 | Öl |
| 109 | $CH{\equiv}C-CH_2-$ | " | 1 | " | Öl |
| 110 | (n) $C_4H_9-$ | " | 1 | " | Öl |

| Bsp. Nr. | $R^1$ | Az | n | $R^2$ | Physikal. Konstante |
|---|---|---|---|---|---|
| 111 | 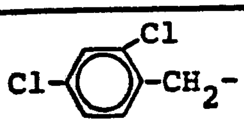 | 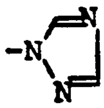 | 1 | 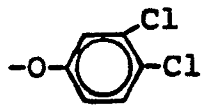 | Öl |
| 112 | H | " | 1 | " | 108-110 |
| 113 | 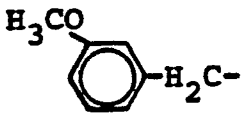 | " | 1 | " | 52-54 |
| 114 | 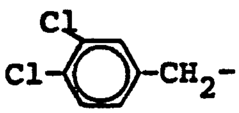 | " | 1 | $-OC_2H_5$ | Öl |
| 115 | 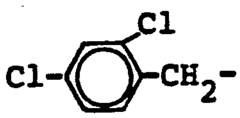 | " | 1 | $-OC_2H_5$ | Öl |
| 116 | $(n)-C_4H_9-$ | " | 1 | 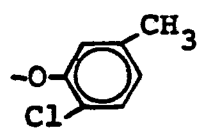 | Öl |
| 117 | $(n)-C_4H_9-$ | " | 1 | 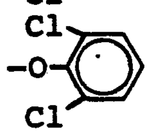 | Öl |
| 118 | 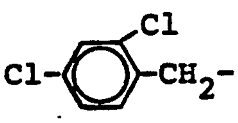 | " | 1 | " | 82-84 |
| 119 | 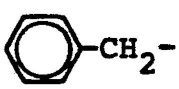 | " | 1 | 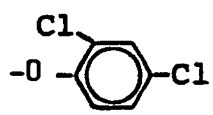 | 48 |
| 120 | 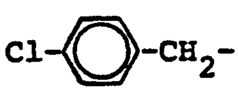 | " | 1 | " | 102 |
| 121 | 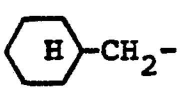 | " | 1 | $-OC_2H_5$ | Öl |
| 122 | 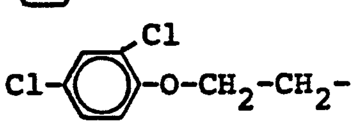 | " | 1 | 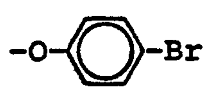 | 36-40 |
| 123 | $(n)-C_4H_9-$ | " | 1 | " | Öl |

| Bsp. Nr. | $R^1$ | Az | n | $R^2$ | Physikal. Konstante |
|---|---|---|---|---|---|
| 124 | 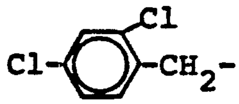 | 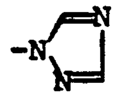 | 1 | 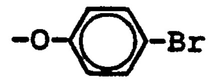 | 145 (x HCl) |
| 125 | " | 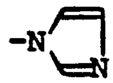 | 1 | 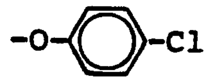 | Öl |
| 126 | 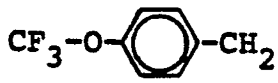 | 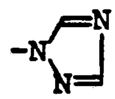 | 1 | 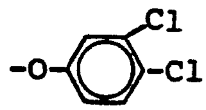 | Öl |
| 127 | $C_2H_5$- | " | 1 | 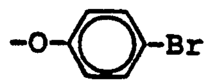 | Öl |
| 128 | H | " | 1 | 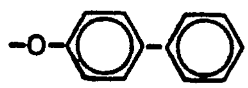 | 62 |
| 129 | H | " | 1 | " | Öl |
| 130 | 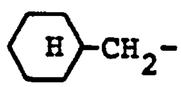 | " | 1 | 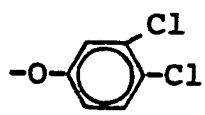 | Öl |
| 131 | H | 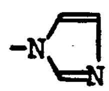 | 1 | 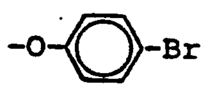 | 46 (x HCl) |
| 132 | (n)-$C_4H_9$- | 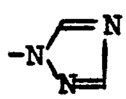 | 1 | 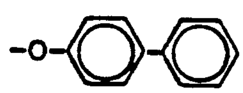 | Öl |
| 133 | (n)-$C_4H_9$- | 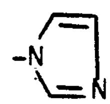 | 1 | " | Öl |
| 134 | 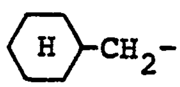 | 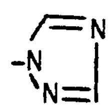 | 0 | 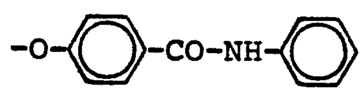 | 93-97 |
| 135 | $CH_2$=CH-$CH_2$- | " | 0 | 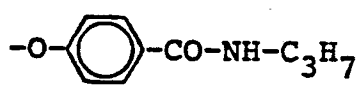 | $n_D^{20}$ = 1,538 |
| 136 | $CH_2$=CH-$CH_2$- | " | 0 | 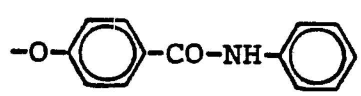 | 116-119 |

| Bsp. Nr. | $R^1$ | Az | n | $R^2$ | Physikal. Konstante |
|---|---|---|---|---|---|
| 137 | H | 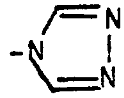 | 1 | 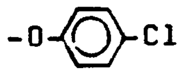 | 124 |
| 138 | H | 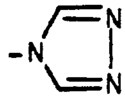 | 1 | $-OCH_3$ | 112 |
| 139 | H | 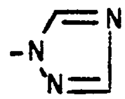 | 1 | 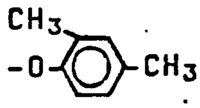 | Oel |
| 140 | H | 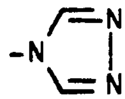 | 1 | 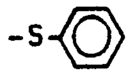 | 110 |
| 141 | $CH_2=CH-CH_2-$ | 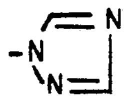 | 0 | 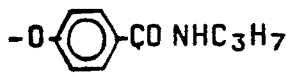 | 1,538 |
| 142 | 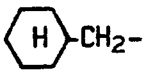 | 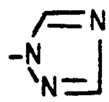 | 0 | 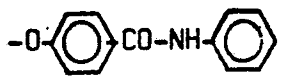 | 93-97 |
| 143 | $CH_2=CH-CH_2-$ | 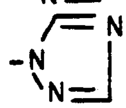 | 0 | 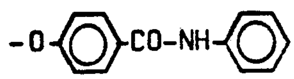 | 116-19 |
| 144 | 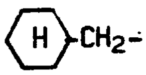 | '' | 1 | 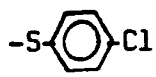 | 1,5600 |
| 145 | $CH_2=CH-CH_2-$ | 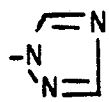 | 1 | 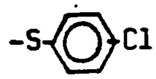 | 1,5686 |
| 146 | $CH\equiv C-CH_2-$ | '' | 1 | 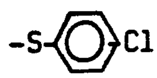 | 1,5771 |
| 147 | 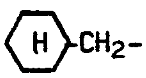 | '' | 1 | 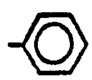 | 50-55 |
| 148 | 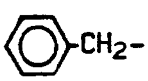 | '' | 1 | 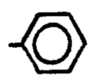 | 45-50 |
| 149 | 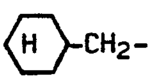 | 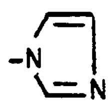 | 1 | 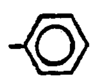 | 1,5383 |
| 150 | 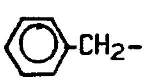 | '' | 1 | 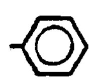 | 1,5677 |

**Anwendungsbeispiele**

In den nachfolgenden Beispielen werden die nachstehend angegebenen Verbindungen als Vergleichssubstanzen eingesetzt:

(A) 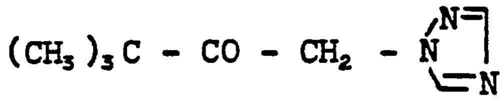

(B) 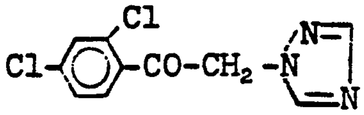

(C) 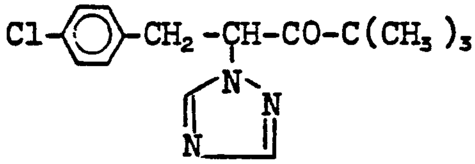

**Beispiel A**

Erysiphe-Test (Gerste) / protektiv
Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Tem-Peratur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender HerstellungsbeisPiele: 43, 28, 29, 22, 3, 44, 24, 1 und 38.

**Beispiel B**

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var hordei bestäubt und bei 21-22°C und 80-90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist. Eine deutliche Ueberlegenheit in der Werksamkeit gegenüber dem Stand der Technik zeigen bei diesem Test z.B. die Verbindungen gemäß folgender Herstellungsbeispiele: 29.

**Beispiel C**

Podosphaera-Test (Apfel) / protektiv
Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkofzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70 % im Gewächshaus. An-schließend werden sie durch Bestäuben mit Konidien des Apfelmehltauerregers (Podosphaera leucotricha) inokuliert und in ein Gewächshaus mit einer Temperatur von 21 bis 23 °C und einer relativen Luftfeuchtigkeit von 70 % gebraucht.

10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Eine deutliche Ueberlegeheit in der Wirksamkeit gegenüber dem Stand der Technik zeigen bei diesem Test z.B. die Verbindungen gemäß folgender Herstellungsbeispiele: 43, 28 und 29.

Wie schon erwähnt, stellen die neuen substituierten 1-Azolyl-butan-2-one der allgemeinen Formel (I) auch interessante Zwischenprodukte dar. Sie lassen sich z.B. leicht in 1-Azolyl-butan-2-ole der Formel

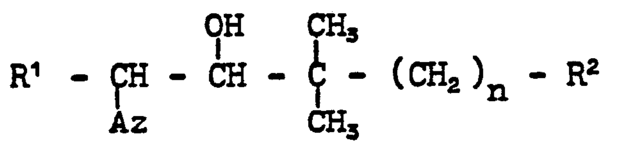

(VIII)

in welcher
$R^1$,$R^2$,Az und n die oben angegebene Bedeutung haben,
überführen, indem man die Verbindungen der Formel (I) nach bekannten Methoden reduziert, wie z.B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30° C.

**Patentansprüche**

1. Substituierte 1-Azolyl-butan-2-one der Formel

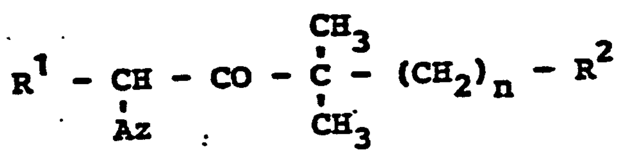

(I)

in welcher
Az für 1,2,4-Triazol-1-yl und -4-yl oder Imidazol-1-yl steht;
$R^1$ für Wasserstoff für Alkyl mit 1 bis 12 und Alkenyl und Alkinyl mit jeweils 2 bis 12 Kohlenstoffatomen steht, für gegebenenfalls durch Alkyl mit 1 bis 4 Kohlenstoffatomen substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im Alkylteil steht, sowie für gegebenenfalls substituiertes Phenoxyalkyl mit 1 bis 4 Kohlenstoffatomen im Alkylteil oder gegebenenfalls substituiertes Phenylalkyl mit 1 bis 4 Kohlenstoffatomen im Alkylteil steht, wobei als Phenyl-Substituenten jeweils zu nennen sind: Halogen; Alkyl, Alkoxy und Alkylthio mit jeweils 1 bis 6 Kohlenstoffatomen; Cyclohexyl; Dialkylamino mit jeweils 1 bis 4 Kohlenstoffatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkylteil; sowie gegebenenfalls durch Halogen substituiertes Phenyl und Phenoxy; sowie die Gruppierung -CO-$NR^6R^7$;
n für die Zahlen 0 oder 1 steht,
$R^2$ für Cyano oder die Gruppierungen -X-$R^3$ und -CO-$NR^4R^5$ steht, sowie für den Fall, daß nicht gleichzeitig n für die Zahl 0, $R^1$ für Wasserstoff und Az für 1,2,4-Triazolyl stehen, noch zusätzlich für gegebenenfalls substituiertes Aryl mit 6 bis 10 Kohlenstoffatomen steht, wobei als Substituenten die bei $R^1$ bereits genannten Arylsubstituenten infrage kommen, sowie für Alkoxycarbonyl mit 1 bis 4 Kohlenstoffatomen im Alkylteil steht;
X für Sauerstoff, Schwefel, die SO- oder $SO_2$-Gruppe steht,

$R^3$ für Alkyl mit 1 bis 6 Kohlenstoffatomen, Halogenalkyl mit 1 bis 4 Kohlenstoffatomen und 1 bis 5 gleichen oder verschiedenen Halogenatomen steht, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im Alkylteil steht, wobei als Substituenten die bei $R^1$ bereits genannten Arylsubstituenten infrage kommen;

$R^4$ für wasserstoff, Alkyl mit 1 bis 4 Kohlenstoffatomen oder gegebenenfalls substituiertes Aryl mit 1 bis 10 Kohlenstoffatomen steht, wobei als Substituenten die bei $R^1$ bereits genannten Arylsubstituenten infrage kommen;

$R^5$ für Wasserstoff oder Alkyl mit 1 bis 4 Kohlenstoffatomen steht; und

$R^6$ und $R^7$ für Wasserstoff, für Alkyl mit 1 bis 4 Kohlenstoffatomen, für gegebenenfalls durch Halogen und Alkyl mit 1 bis 4 Kohlenstoffatomen substituiertes Phenyl stehen, oder beide zusammen mit dem angrenzenden Stickstoffatom für ein gesättigtes 5- oder 6-gliedriges Ringsystem stehen, welches Stickstoff oder Sauerstoff als zusätzliche Heteroatome enthalten kann;

sowie deren pflanzenverträgliche Säureadditions-Salze und Metallsalz-Komplexe.

2. Substituierte 1-Azolyl-butan-2-one der Formel (I) in Anspruch 1,

in welcher

$R^1$ für geradkettiges oder verzweigtes Alkyl mit 1 bis 6 Kohlenstoffatomen, Alkenyl und Alkynyl mit jeweils 2 bis 6 Kohlenstoffatomen, gegebenenfalls durch Methyl substituiertes Cyclohexyl und Cyclohexylmethyl, sowie für gegebenenfalls substituiertes Phenoxyalkyl und gegebenenfalls substituiertes Phenylalkyl mit jeweils 1 bis 2 Kohlenstoffatomen im Alkylteil steht, wobei als Phenyl-Substituenten genannt seien: Fluor, Chlor, Methyl, Ethyl, Isopropyl, tert.-Butyl, Dimethylamino, Methoxy, Methylthio, Cyclohexyl, Trifluormethyl, Trifluormethoxy, Trifluormethylthio, Nitro, Cyano, sowie gegebenenfalls durch Fluor und Chlor substituiertes Phenyl und Phenoxy, sowie die Morpholinocarbonyl-, Phenylaminocarbonyl-, Chlorphenylaminocarbonyl- und Dibutylaminocarbonyl-Gruppe;

$R^2$ für Cyano; die Gruppierungen $-X-R^3$ und $-CO-NR^4R^5$ steht; sowie für den Fall, daß nicht gleichzeitig n für die Zahl O, $R^1$ für Wasserstoff und Az für 1,2,4-Triazol stehen, noch für gegebenenfalls substituiertes Phenyl steht, wobei als Substituenten die bei $R^1$ bereits genannten Phenylsubstituenten infrage kommen und schließlich für Methoxycarbonyl, Ethoxycarbonyl und Isopropoxycarbonyl steht;

$R^3$ für geradkettiges oder verzweigtes Alkyl mit 1 bis 4 Kohlenstoffatomen oder gegebenenfalls substituiertes Phenyl und Benzyl steht, wobei als Substituenten an den jeweiligen Phenylresten die bei $R^1$ bereits genannten Phenylsubstituenten infrage kommen;

$R^4$ für Wasserstoff, Methyl, Ethyl, Isopropyl oder gegebenenfalls substituiertes Phenyl steht, wobei als Substituenten Halogen und Alkyl mit 1 bis 4 Kohlenstoffatomen infrage kommen;

$R^5$ für Wasserstoff, Methyl, Ethyl oder Isopropyl steht; und

Az, X und der Index n die im Anspruch 1 angegebene Bedeutung haben.

3. Verfahren zur Herstellung von substituierten 1-Azolyl-butan-2-onen der Formel

$$R^1 - \underset{\substack{|\\ Az}}{CH} - CO - \overset{\substack{CH_3\\ |}}{\underset{\substack{|\\ CH_3}}{C}} - (CH_2)_n - R^2 \qquad (I)$$

in welcher

Az für 1,2,4-Triazol-1-yl und -4-yl oder Imidazol-1-yl steht;

$R^1$ für Wasserstoff, für Alkyl mit 1 bis 12 und Alkenyl und Alkinyl mit jeweils 2 bis 12 Kohlenstoffatomen steht, für gegebenenfalls durch Alkyl mit 1 bis 4 Kohlenstoffatomen substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im Alkylteil steht, sowie für gegebenenfalls substituiertes Phenoxyalkyl mit 1 bis 4 Kohlenstoffatomen im Alkylteil oder gegebenenfalls substituiertes Phenylalkyl mit 1 bis 4 Kohlenstoffatomen im Alkylteil steht, wobei als Phenyl-Substituenten jeweils zu nennen sind: Halogen; Alkyl, Alkoxy und Alkylthio mit jeweils 1 bis 6 Kohlenstoffatomen; Cyclohexyl; Dialkylamino mit jeweils 1 bis 4 Kohlenstoffatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkylteil; sowie gegebenenfalls durch Halogen substituiertes Phenyl und Phenoxy; sowie die Gruppierung $-CO-NR^6R^7$;

n für die Zahlen 0 oder 1 steht,

$R^2$ für Cyano oder die Gruppierungen $-X-R^3$ und $-CO-NR^4R^5$ steht, sowie für den Fall, daß nicht gleichzeitig n für die Zahl 0, $R^1$ für Wasserstoff und Az für 1,2,4-Triazolyl stehen, noch zusätzlich für gegebenenfalls substituiertes Aryl mit 6 bis 10 Kohlenstoffatomen steht, wobei als Substituenten die bei $R^1$ bereits genannten Arylsubstituenten infrage kommen, sowie für Alkoxycarbonyl mit 1 bis 4 Kohlenstoffatomen im Alkylteil steht;

X für Sauerstoff, Schwefel, die SO- oder SO2-Gruppe steht,

$R^3$ für Alkyl mit 1 bis 6 Kohlenstoffatomen, Halogenalkyl mit 1 bis 4 Kohlenstoffatomen und 1 bis 5 gleichen oder verschiedenen Halogenatomen steht, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im

Alkylteil steht, wobei als Substituenten die bei $R^1$ bereits genannten Arylsubstituenten infrage kommen;

$R^4$ für Wasserstoff, Alkyl mit 1 bis 4 Kohlenstoffatomen oder gegebenenfalls substituiertes Aryl mit 1 bis 10 Kohlenstoffatomen steht, wobei als Substituenten die bei $R^1$ bereits genannten Arylsubstituenten infrage kommen;

$R^5$ für Wasserstoff oder Alkyl mit 1 bis 4 Kohlenstoffatomen steht; und

$R^6$ und $R^7$ für Wasserstoff, für Alkyl mit 1 bis 4 Kohlenstoffatomen, für gegebenenfalls durch Halogen und Alkyl mit 1 bis 4 Kohlenstoffatomen substituiertes Phenyl stehen, oder beide zusammen mit dem angrenzenden Stickstoffatom für ein gesättigtes 5- oder 6-gliedriges Ringsystem stehen, welches Stickstoff oder Sauerstoff als zusätzliche Heteroatome enthalten kann;

dadurch gekennzeichnet, daß man

a) Halogenketone der Formel

$$Hal - CH_2 - CO - \underset{CH_3}{\overset{CH_3}{C}} - (CH_2)_n - R^2 \qquad (II)$$

in welcher

Hal für Halogen steht und

$R^2$ und n die oben angegebene Bedeutung haben, wobei jedoch in der Gruppierung -X-$R^3$ der Substituent X nur für Sauerstoff oder Schwefel steht,

mit Azolen der Formel

H - Az (III)

in welcher

Az die oben angegebene Bedeutung hat,

in Gegenwart eines Verdünnungsmittels und in Gegenwart eines Säurebindemittels umsetzt; und gegebenenfalls

b) die so erhaltenen Verbindungen der Formel

$$Az - CH_2 - CO - \underset{CH_3}{\overset{CH_3}{C}} - (CH_2)_n - R^2 \qquad (Ia)$$

in welcher

Az, n und $R^2$ die oben angegebene Bedeutung haben,

mit einem Alkylierungsmittel der Formel

$R^1$ - Z (IV)

in welcher

$R^1$ die oben angegebene Bedeutung hat und

Z für eine elektronenanziehende Abgangsgruppierung steht, in Gegenwart einer Base und in Gegenwart eines organischen Verdünnungsmittels, oder in einem wäßrig-organischen ZweiPhasensystem in Gegenwart eines Phasentransferkatalysators, umsetzt; und gegebenenfalls

c) die nach den Verfahren (a) und (b) erhaltenen Verbindungen der Formel

$$R^1 - \underset{Az}{CH} - CO - \underset{CH_3}{\overset{CH_3}{C}} - (CH_2)_n - S - R^3 \qquad (Ib)$$

in welcher

Az, $R^1$, n und $R^3$ die oben angegebene Bedeutung haben,

nach bekannten Methoden in üblicher Weise oxidiert,

und an die so erhaltenen Verbindungen der Formel (I) gegebenenfalls anschließend noch eine Säure oder ein Metallsalz addiert.

4. Fungizide Mittel, gekennzeichnet durch einen Gehalt an mindestens einem substituierten 1-Azolyl-butan-2-on der Formel (I) in Ansprüchen 1 und 3.

5. Verwendung von substituierten 1-Azolyl-butan-2-onen der Formel (I) in Ansprüchen 1 und 3 zur Bekämpfung von phytopathogenen Pilzen.

6. Verfahren zur Herstellung von fungiziden Mitteln, dadurch gekennzeichnet, daß man substituierte 1-Azolyl-butan-2-one der Formel (I) in den Ansprüchen 1 und 3 mit Streckmitteln und/oder oberflächenaktiven Mitteln

vermischt.

## Claims

1. Substituted 1-azolyl-butan-2-ones of the formula

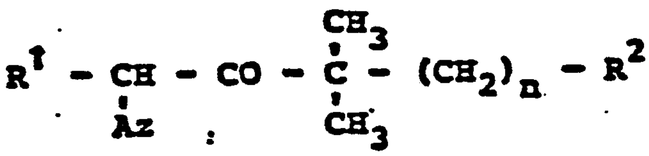

(I)

in which

Az represents 1,2,4-triazol-1-yl and -4-yl or imidazol-1-yl; $R^1$ represents hydrogen, alkyl with 1 to 12 and alkenyl and alkinyl with in each case 2 to 12 carbon atoms,and represents optionally $C_1$-$C_4$-alkyl-substituted cycloalkyl with 3 to 7 carbon atoms or cycloalkylalkyl with 3 to 7 carbon atoms in the cycloalkyl part and 1 to 4 carbon atoms in the alkyl part, and represents optionally substituted phenoxyalkyl with 1 to 4 carbon atoms in the alkyl part or optionally substituted phenylalkyl with 1 to 4 carbon atoms in the alkyl part, the phenyl substituents to be mentioned in each case being: halogen; alkyl, alkoxy and alkylthio with in each case 1 to 6 carbon atoms; cyclohexyl; dialkylamino with in each case 1 to 4 carbon atoms in each alkyl part; halogenoalkyl, halogenoalkoxy and halogenoalkylthio with in each case 1 to 4 carbon atoms and 1 to 5 identical or different halogen atoms; nitro; cyano; alkoxycarbonyl with 1 to 4 carbon atoms in the alkyl part; and optionally halogensubstituted phenyl and phenoxy; and the grouping -CO-$NR^6R^7$; n represents the numbers 0 or 1,

$R^2$ represents cyano or the groupings -X-$R^3$ and -CO-$NR^4R^5$, and, in the case where n does not represent O at the same time as $R^1$ represents hydrogen and Az represents 1,2,4-triazolyl, also additionally represents optionally substituted aryl with 6 to 10 carbon atoms, possible substituents being the aryl substituents already mentioned under $R^1$, and represents alkoxycarbonyl with 1 to 4 carbon atoms in the alkyl part; X represents oxygen, sulphur, or the SO or $SO_2$ group, $R^3$ represents alkyl with 1 to 6 carbon atoms, halogenoalkyl with 1 to 4 carbon atoms and 1 to 5 identical or different halogen atoms, and represents optionally substituted aryl with 6 to 10 carbon atoms and aralkyl with 6 to 10 carbon atoms in the aryl part and 1 to 2 carbon atoms in the alkyl part, possible substituents being the aryl substituents already mentioned under $R^1$;

$R^4$ represents hydrogen, alkyl with 1 to 4 carbon atoms or optionally substituted aryl with 1 to 10 carbon atoms, possible substituents being the aryl substituents already mentioned under $R^1$;

$R^5$ represents hydrogen or alkyl with 1 to 4 carbon atoms; and $R^6$ and $R^7$ represent hydrogen, alkyl with 1 to 4 carbon atoms, phenyl which is optionally substituted by halogen and alkyl with 1 to 4 carbon atoms, or both together with the adjacent nitrogen atom represent a saturated 5- or 6-membered ring system which can contain nitrogen or oxygen as additional hetero atoms;

and plant-tolerated acid addition salts and metal salt complexes thereof.

2. Substituted 1-azolyl-butan-2-ones of the formula (I) in Claim 1,

in which

$R^1$ represents straight-chain or branched alkyl with 1 to 6 carbon atoms, alkenyl and alkinyl with in each case 2 to 6 carbon atoms, optionally methyl-substituted cyclohexyl and cyclohexylmethyl, and optionally substituted phenoxyalkyl and optionally substituted phenylalkyl with in each case 1 to 2 carbon atoms in the alkyl part, the following being mentioned as the phenyl substituents: fluorine, chlorine, methyl, ethyl, isopropyl, tert.-butyl, dimethylamino, methoxy, methylthio, cyclohexyl, trifluoromethyl, trifluoromethoxy, trifluoromethylthio, nitro, cyano, and optionally fluorineand chlorine-substituted phenyl and phenoxy, and the morpholinocarbonyl, phenylaminocarbonyl, chlorophenylaminocarbonyl and dibutylaminocarbonyl group;

$R^2$ represents cyano; the groupings -X-$R^3$ and -CO-$NR^4R^5$, and in the case where n does not represent the number 0 at the same time as $R^1$ represents hydrogen and Az represents 1,2,4-triazole, also represents optionally substituted phenyl, possible substituents being the phenyl substituents already mentioned under $R^1$, and finally represents methoxycarbonyl, ethoxycarbonyl and isopropoxycarbonyl;

$R^3$ represents straight-chain or branched alkyl with 1 to 4 carbon atoms or optionally substituted phenyl and benzyl, possible substituents on the particular phenyl radicals being the phenyl substituents already mentioned under $R^1$;

$R^4$ represents hydrogen, methyl, ethyl, isopropyl or optionally substituted phenyl, possible substituents being halogen and alkyl with 1 to 4 carbon atoms;

$R^5$ represents hydrogen, methyl, ethyl or isopropyl; and Az, X and the index n have the meaning given in Claim 1.

3. Process for the preparation of substituted 1 azolyl-butan-2-ones of the formula

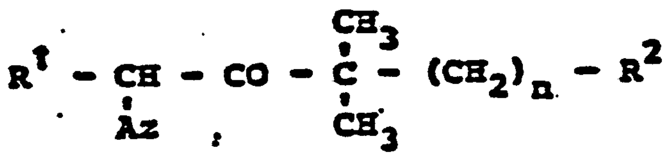 (I)

in which

Az represents 1,2,4-triazol-1-yl and -4-yl or imidazol-1-yl; $R^1$ represents hydrogen, alkyl with 1 to 12 and alkenyl and alkiny with in each case 2 to 12 carbon atoms, and represents optionally $C_1$-$C_4$-alkyl-substituted cycloalkyl with 3 to 7 carbon atoms or cycloalkylalkyl with 3 to 7 carbon atoms in the cycloalkyl part and 1 to 4 carbon atoms in the alkyl part,and representsoptionally substituted phenoxyalkyl with 1 to 4 carbon atoms in the alkyl part or optionally substituted phenylalkyl with 1 to 4 carbon atoms in the alkyl part, the phenyl substituents to be mentioned in each case being: halogen; alkyl, alkoxy and alkylthio with in each case 1 to 6 carbon atoms; cyclohexyl; dialkylamino with in each case 1 to 4 carbon atoms in each alkyl part; halogenoalkyl, halogenoalkoxy and halogenoalkylthio with in each case 1 to 4 carbon atoms and 1 to 5 identical or different halogen atoms; nitro; cyano; alkoxycarbonyl with 1 to 4 carbon atoms in the alkyl part; and optionally halogensubstituted phenyl and phenoxy; and the grouping -CO-$NR^6R^7$; n represents the numbers 0 or 1,

$R^2$ represents cyano or the groupings -X-$R^3$ and -CO-$NR^4R^5$, and, in the case where n doesnot represent 0 at the same time as $R^1$ represents hydrogen and Az represents 1,2,4-triazolyl, also additionally represents optionally substituted aryl with 6 to 10 carbon atoms, possible substituents being the aryl substituents already mentioned under $R^1$, and represents alkoxycarbonyl with 1 to 4 carbon atoms in the alkyl part; X represents oxygen, sulphur, or the SO or SO2 group, $R^3$ represents alkyl with 1 to 6 carbon atoms, halogenoalkyl with 1 to 4 carbon atoms and 1 to 5 identical or different halogen atoms, and represents optionally substituted aryl with 6 to 10 carbon atoms and aralkyl with 6 to 10 carbon atoms in the aryl part and 1 to 2 carbon atoms in the alkyl part, possible substituents being the aryl substituents already mentioned under $R^1$;

$R^4$ represents hydrogen, alkyl with 1 to 4 carbon atoms or optionallyubstituents substituted aryl with 1 to 10 carbon atoms, possible substitents being the aryl substituents already mentioned under $R^1$;

$R^5$ represents hydrogen or alkyl with 1 to 4 carbon atoms; and $R^6$ and $R^7$ represent hydrogen, alkyl with 1 to 4 carbon atoms, phenyl which is optionally substituted by halogen and alkyl with 1 to 4 carbon atoms, or both together with the adjacent nitrogen atom represent a saturated 5- or 6-membered ring system which can contain nitrogen or oxygen as additional hetero atoms;

characterised in that

a) halogenoketones of the formula

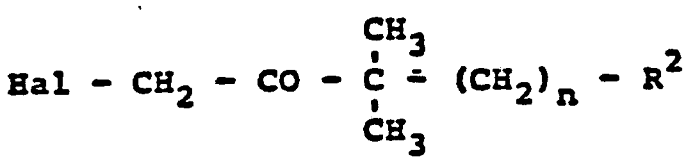 (II)

in which

Hal represents halogen and

$R^2$ and n have the meaning given above, but in the grouping -X-$R^3$ the substituent X only represents oxygen or sulphur, are reacted with azoles of the formula

H - Az (III)

in which

Az has the meaning given above,

in the presence of a diluent and in the presence of an acid-binding agent; and, if desired,

b) the compounds thus obtained, of the formula

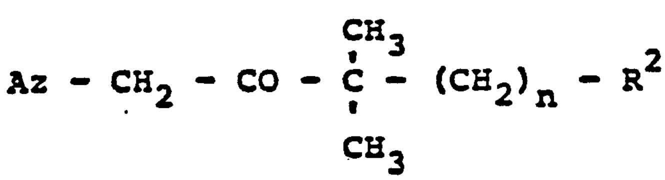 (Ia)

in which

Az, n and $R^2$ have the meaning given above,

are reacted with an alkylating agent of the formula

R - Z - (IV)

in which

$R^1$ has the meaning given above and

Z represents an electron-attracting leaving group, in the presence of a base and in the presence of an organic diluent, or in an aqueous-organic two-phase system in the presence of a phase transfer catalyst; and, if desired,

c) the compounds obtained by reaction variants (a) and (b), of the formula

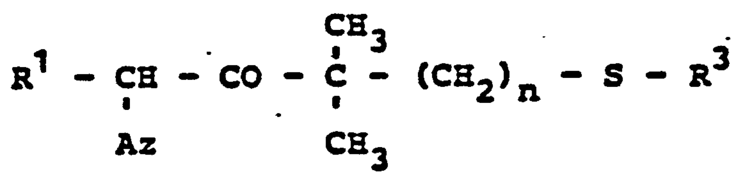

(Ib)

in which

Az, $R^1$, n and $R^3$ have the meaning given above, are oxidised in a customary manner by known methods, and, if desired, an acid or a metal salt is then added on to the compounds thus obtained, of the formula (I).

4. Fungicidal agents, characterised in that they contain at least one substituted 1-azolyl-butan-2-one of the formula (I) in Claims 1 and 3.

5. Use of substituted 1-azolyl-butan-2-ones of the formula (I) in Claims 1 and 3 for combating phytopathogenic fungi.

6. Process for preparing fungicidal agents, characterised in that substituted 1-azolyl-butan-2-ones of the formula (I) in Claims 1 and 3 are mixed with extenders and/or surface-active agents.

**Revendications**

1. 1-azolyl-butane-2-ones substituées, de formule

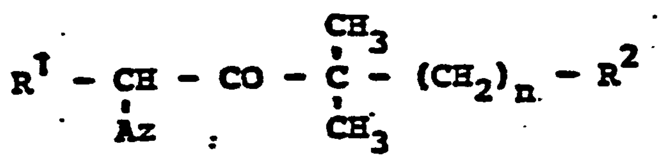

(I)

dans laquelle

Az représente un groupe 1,2,4-triazole-1-yle et 4-yle ou imidazole-1-yle;

$R^1$ est l'hydrogène, un groupe alkyle ayant 1 à 12 atomes de carbone et des groupes alcényle et alcynyle ayant chacun 2 à 12 atomes de carbone, un groupe cycloalkyle ayant 3 à 7 atomes de carbone éventuellement substitué par un radical alkyle ayant 1 à 4 atomes de carbone, ou un groupe cycloalkylalkyle ayant 3 à 7 atomes de carbone dans la partie cycloalkyle et 1 à 4 atomes de carbone dans la partie alkyle, ainsi qu'un groupe phénoxyalkyle éventuellement substitué ayant 1 à 4 atomes de carbone dans la partie alkyle ou un groupe phénylalkyle éventuellement substitué ayant 1 à 4 atomes de carbone dans la partie alkyle et on peut alors mentionner dans chaque cas comme substituants du groupe phényle: un halogéne; des substituants alkyle, alkoxy et alkylthio ayant chacun 1 à 6 atomes de carbone; un substituant cyclohexyle; dialkylamino ayant 1 à 4 atomes de carbone dans chaque partie alkyle; des substituants halogénalkyle, halogénalkoxy et halogénalkylthio ayant chacun 1 à 4 atomes de carbone et 1 à 5 atomes d'halogènes égaux ou différents; nitro; cyano; alkoxycarbonyle ayant 1 à 4 atomes de carbone dans la partie alkyle; ainsi qu'un substituant phényle et un substituant phénoxy éventuellement substitués par un halogène) de même que le groupement -CO-$NR^6R^7$;,

n représente les nombres 0 ou 1,

$R^2$ est un groupe cyano ou représente les groupements -X-$R^3$ et -CO-$NR^4R^5$, de méme que, au cas où n n'a pas la valeur 0 en même temps que $R^1$ représente l'hydrogène et que Az est un groupe 1,2,4-triazolyle, $R^2$ représente en outre un groupe aryle de 6 à 10 atomes de carbone éventuellement substitué, et on considère alors comme substituants les substituants d'aryle déjà mentionnés dans le cas de $R^1$ ainsi qu'un groupe alkoxycarbonyle ayant 1 à 4 atomes de carbone dans la partie alkyle;

X représente l'oxygéne, le soufre, le groupe SO- ou le groupe $SO_2$-,

$R^3$ est un groupe alkyle ayant 1 à 6 atomes de carbone, halogénalkyle ayant 1 à 4 atomes de carbone et 1 à 5 atomes d'halogènes égaux ou différents, ainsi qu'un groupe aryle de 6 à 10 atomes de carbone et un groupe aralkyle de 6 à 10 atomes de carbone dans la partie aryle et de 1 ou 2 atomes de carbone dans la partie alkyle, éventuellement substitués, et on considére alors comme substituants les substituants d'aryle déjà mentionnés dans le cas de $R^1$;

$R^4$ représente l'hydrogène, un groupe alkyle ayant 1 à 4 atomes de carbone ou un groupe aryle éventuellement substitué ayant 1 à 10 atomes de carbone, et on considère alors comme substituants les substituants d'aryle déjà mentionnés à propos de $R^1$;

$R^5$ représente l'hydrogène ou un groupe alkyle ayant 1 à 4 atomes de carbone; et

$R^6$ et $R^7$ représentent l'hydrogène, un groupe alkyle ayant 1 à 4 atomes de carbone, un groupe phényle éventuellement substitué par un halogène et un substituant alkyle ayant 1 à 4 atomes de carbone, ou bien tous deux s'associent pour former avec l'atome adjacent d'azote un noyau pentagonal ou hexagonal saturé qui peut comporter de l'azote ou de l'oxygène comme hétéroatomes supplémentaires;

ainsi que leur sels d'addition d'acides et leurs complexes de sels métalliques compatibles avec les végétaux.

2. 1-azolyl-butane-2-ones substituées de formule (I) suivant la revendication 1,

dans laquelle

$R^1$ désigne un groupe alkyle à chaîne droite ou ramifié ayant à 1 à 6 atomes de carbone, un groupe alcényle et un groupe alcynyle ayant chacun 2 à 6 atomes de carbone, un groupe cyclohexyle et un groupe cyclohexylméthyle éventuellement substitués par un radical méthyle, ainsi qu'un groupe phénoxyalkyle éventuellement substitué et un groupe phénylalkyle éventuellement substitué avec dans chaque cas un ou deux atomes de carbone dans la partie alkyle, et on mentionne alors comme substituants du groupe phényle-. le fluor, le chlore, les radicaux méthyle, éthyle, isopropyle, tertio-butyle, diméthylamino, méthoxy, méthylthio, cyclohexyle, trifluorométhyle, trifluorométhoxy, trifluorométhylthio, nitro, cyano, ainsi que les substituants phényle et phénoxy éventuellement substitués par du fluor et du chlore, de même que les groupes morpholinocarbonyle, phénylaminocarbonyle, chlorophénylaminocarbonyle et dibutylaminocarbonyle;

$R^2$ représente le groupe cyano; les groupements -X-R3 et $-CO-NR^4R^5$; ainsi que, au cas où n n'a pas la valeur 0 en même temps que $R^1$ représente l'hydrogène et que Az est un groupe 1,2,4-triazole, $R^2$ représente en outre un groupe phényle éventuellement substitué et on considère alors comme substituants les substituants du groupe phényle déjà mentionnés pour $R^1$, enfin, les groupes méthoxycarbonyle, éthoxycarbonyle et isopropoxycarbonyle;

$R^3$ est un groupe alkyle à chaîne droite ou à chaîne ramifiée ayant 1 à 4 atomes de carbone ou les groupes phényle et benzyle éventuellement substitués, et on considère alors comme substituants sur les restes phényle correspondants les substituants du groupe phényle déjà mentionnés dans le cas de $R^1$,

$R^4$ représente l'hydrogène, un groupe méthyle, éthyle, isopropyle, ou un groupe phènyle éventuellement substitué, et on considère alors comme substituants un halogène et un substituant alkyle ayant 1 à 4 atomes de carbone,

$R^5$ est l'hydrogène, un groupe méthyle, éthyle ou isopropyle; et

Az, X et

l'indice n ont la définition indiquée dans la revendication 1.

3. Procédé de production de 1-azolyl-butane-2-ones substituées de formule

$$R^1-\underset{\displaystyle Az}{\underset{|}{CH}}-CO-\overset{\displaystyle CH_3}{\overset{|}{\underset{\displaystyle CH_3}{\underset{|}{C}}}}-(CH_2)_n-R^2 \qquad (I)$$

dans laquelle

Az représente un groupe 1,2,4-triazole-1-yle et 4-yle ou imidazole-1-yle;

$R^1$ est l'hydrogène, un groupe alkyle ayant 1 à 12 atomes de carbone et des groupes alcényle et alcynyle ayant chacun 2 à 12 atomes de carbone, un groupe cycloalkyle ayant 3 à 7 atomes de carbone éventuellement substitué par un radical alkyle ayant 1 à 4 atomes de carbone, ou un groupe cycloalkylalkyle ayant 3 à 7 atomes de carbone dans la partie cycloalkyle et 1 à 4 atomes de carbone dans la partie alkyle, ainsi qu'un groupe phénoxyalkyle éventuellement substitué ayant 1 à 4 atomes de carbone dans la partie alkyle ou un groupe phénylalkyle éventuellement substitué ayant 1 à 4 atomes de carbone dans la partie alkyle et on peut alors mentionner dans chaque cas comme substituants du groupe phényle: un halogène; des substituants alkyle, alkoxy et alkylthio ayant chacun 1 à 6 atomes de carbone; un substituant cyclohexyle; dialkylamino ayant 1 à 4 atomes de carbone dans chaque partie alkyle; des substituants halogénalkyle, halogénalkoxy et halogénalkylthio ayant chacun 1 à 4 atomes de carbone et 1 à 5 atomes d'halogènes égaux ou différents; nitro; cyano, alkoxycarbonyle ayant 1 à 4 atomes de carbone dans la partie alkyle; ainsi qu'un substituant phényle et un substituant phénoxy éventuellement substitués par un halogène) de même que le groupement $-CO-NR^6R^7$;,

n représente les nombres 0 ou 1,

$R^2$ est un groupe cyano ou représente les groupements $-X-R^3$ et $-CO-NR^4R^5$, de même que, au cas où n n'a pas la valeur 0 en même temps que $R^1$ représente l'hydrogéne et que Az est un groupe 1,2,4-triazolyle, $R^2$ représente en outre un groupe aryle de 6 à 10 atomes de carbone éventuellement substitué, et on considére alors comme substituants les substituants d'aryle déjà mentionnés dans le cas de $R^1$ ainsi qu'un groupe alkoxycarbonyle ayant 1 à 4 atomes de carbone dans la partie alkyle;

X représente l'oxygène, le soufre, le groupe SO- ou le groupe $SO_2$-,

$R^3$ est un groupe alkyle ayant 1 à 6 atomes de carbone, halogénalkyle ayant 1 à 4 atomes de carbone et 1 à 5 atomes d'halogènes égaux ou différents, ainsi qu'un groupe aryle de 6 à 10 atomes de carbone et un groupe aralkyle de 6 à 10 atomes de carbone dans la partie aryle et de 1 ou 2 atomes de carbone dans la partie alkyle, éventuellement substitués, et on considére alors comme substituants les substituants d'aryle déjà mentionnés dans le cas de $R^1$;

$R^4$ représente l'hydrogène, un groupe alkyle ayant 1 à 4 atomes de carbone ou un groupe aryle éventuellement

substitué ayant 1 à 10 atomes de carbone, et on considére alors comme substituants les substituants d'aryle dejà mentionnés à propos de $R^1$.

$R^5$ représente l'hydrogène ou un groupe alkyle ayant 1 à 4 atomes de carbone; et

$R^6$ et $R^7$ représentent l'hydrogène, un groupe alkyle ayant 1 à 4 atomes de carbone, un groupe phényle éventuellement substitué par un halogène et un substituant alkyle ayant 1 à 4 atomes de carbone, ou bien tous deux s'associent pour former avec l'atome adjacent d'azote un noyau pentagonal ou hexagonal saturé qui peut comporter de l'azote ou de l'oxygène comme hétéroatomes supplémentaires,

caractérisé en ce que:

a) on fait réagir des halogénocétones de formule

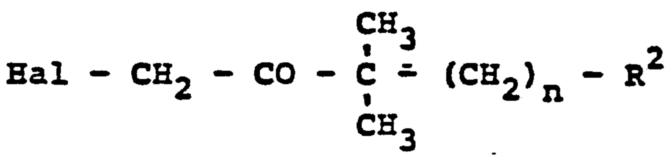 (II)

dans laquelle

Hal représente un halogène et

$R^2$ et n ont la définition indiquée ci-dessus, sous réserve que dans le groupement -X-$R^3$, le substituant X ne représente que l'oxygène ou le soufre, avec des azoles de formule

H - Az (III)

dans laquelle

Az a la définition indiquée cidessus, en présence d'un diluant et en présence d'un accepteur d'acide; et le cas échéant,

b) on fait réagir les composés ainsi obtenus de formule

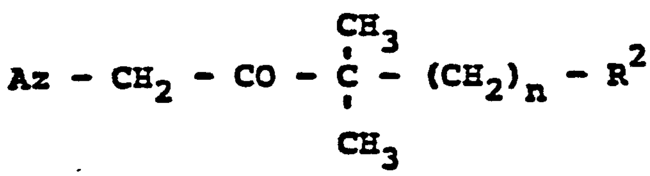 (Ia)

dans laquelle

Az, n et $R^2$ ont la définition indiquée ci-dessus,

avec un agent alkylant de formule

$R^1$ - Z (IV)

dans laquelle

$R^1$ a la définition indiquée ci-dessus et

Z est un groupement partant attirant les électrons,

en présence d'une base et en présence d'un diluant organique ou dans un système aqueuxorganique de deux phases en présence d'un catalyseur de transfert de phases; et le cas échéant

c) on oxyde d'une manière classique par des procédés connus les composés obtenus selon les procédés (a) et (b), de formule

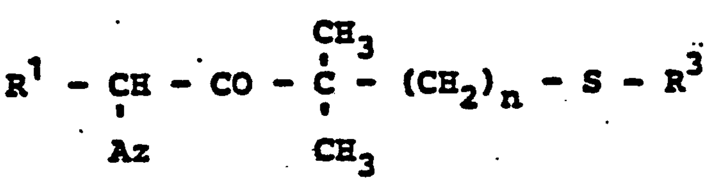 (Ib)

dans laquelle

Az, $R^1$, n

et $R^3$ ont la définition indiquée ci-dessus, puis on additionne encore éventuellement un acide ou un sel métallique sur les composés ainsi obtenus, de formule (I).

4. Compositions fongicides, caractérisées par une teneur en au moins une 1-azolyl-butane-2-one substituée de formule (I) suivant les revendications 1 et 3.

5. Utilisation de 1-azolyl-butane-2-ones substituées de formule (I) suivant les revendications 1 et 3 pour combattre des champignons phytophathogènes.

6. Procédé de préparation de compositions fongicides, caractérisé en ce qu'on mélange des 1-azolyl-butane-2-ones substituées de formule (I) suivant les revendications 1 et 3 avec des diluants et/ou des agents tensio-actifs.